# EUROPEAN PATENT SPECIFICATION

(11) **EP 2 692 779 B1**
(45) Date of publication and mention of the grant of the patent: **25.01.2017**
(21) Application number: 12764002.7
(22) Date of filing: 30.01.2012
(51) Int. Cl.: C08J 3/12, A61K 8/02, A61K 8/81, C08G 61/12, C08J 7/04, C08L 65/00, C09D 165/00, G02B 1/10

(54) **COLORED RESIN PARTICLE, METHOD FOR PRODUCING SAME, AND USE THEREOF**
GEFÄRBTE HARZTEILCHEN, VERFAHREN ZU IHRER HERSTELLUNG UND VERWENDUNG DAVON
PARTICULE DE RÉSINE COLORÉE, SON PROCÉDÉ DE FABRICATION ET SON UTILISATION

(30) Priority: 31.03.2011 JP 2011078826
(43) Date of publication of application: 05.02.2014
(73) Proprietor: Sekisui Plastics Co., Ltd., Osaka-shi Osaka 530-8565 (JP)
(72) Inventor: NAKAMURA Masaaki, Koka-shi, Shiga 528-0056 (JP); HARADA Ryosuke, Koka-shi, Shiga 528-0056 (JP)
(74) Representative: Müller-Boré & Partner Patentanwälte PartG mbB
(86) International application number: PCT/JP2012/051967
(87) International publication number: WO 2012/132533

(56) References cited:
- EP-A1- 0 460 430
- EP-A1- 1 209 530
- WO-A1-2006/015756
- JP-A- 4 041 410
- JP-A- 2003 086 020
- JP-A- 2003 086 020
- JP-A- 2004 241 132
- JP-A- 2004 241 132
- JP-A- 2007 254 558
- JP-A- 2008 508 411
- JP-A- 2012 072 261
- JP-A- 2012 072 264
- US-A1- 2010 189 956

## Description

The present invention relates to colored resin particles and a method for producing the same, and more specifically relates to colored resin particles surface-coated with a polymer derived from a nitrogen-containing aromatic compound, a method for producing the same, and use thereof (resin compositions, molded bodies, light-diffusing plates, coating materials, coated products, optical films, anti-glare films, and external preparations).

Conventionally, in the field of cosmetic materials, there is a known technique in which fine particles made of synthetic macromolecules and surface-coated with black pyrrole are used as a black pigment, in order to provide a cosmetic material composition that is harmless to the human body and that contains a pigment (black pigment) having a high blackness (see Patent Document 1 below). Further, JP 2003-086020 A discloses conductive particles which are provided with a conductive film coating the particle main body, wherein the conductive film is composed of an oxidation polymerization reactant of aromatic compound. JP 2004-241132 A discloses a conductive paint composition in which a polypyrrole layer is provided on the surface of sheet particulate and resin emulsion particles. WO 2006/015756 A1 discloses a method for coating metallic surfaces with an anti-corrosive composition that contains a conductive polymer, wherein the conductive polymer is mainly or entirely in particulate form. EP 1 209 530 A1 discloses a toner comprising particles of a resin and an optional colorant, wherein said toner particles have a polypyrrole-coating.
[Patent Document 1] JP H4-41410A
[Patent Document 2] International Publication No. 2008/018339

However, in Patent Document 1, no research was conducted on the shape of the fine particles coated with black pyrrole, and ordinary spherical fine particles are used. Black particles obtained using spherical fine particles are spherical, and thus, the rolling friction is small, and the adhesiveness is poor.

Meanwhile, display apparatuses such as liquid crystal displays may have a problem in which, when their surroundings are bright, external light is specularly reflected on the display face like a mirror, which makes it difficult to see information such as images or video displayed on the display face. In order to solve such a problem, a technique has been adopted in which the display face is provided with a anti-glare film to diffuse light reflected on the display face, so that the display face is made anti-glare, and specular reflection of external light due to surface reflection is reduced.

A conventional anti-glare film is configured such that minute roughness on its surface provides anti-glare properties to a display face of a display apparatus. As a method for forming minute roughness, a method in which a dispersion obtained by dispersing resin particles in a binder resin is applied over a film and is then dried to form a coating film thereon is mainly used because the roughness can be easily adjusted and the production can be efficiently performed.

However, this conventional anti-glare film is problematic in that the anti-glare layer (the coating film in which the resin particles are dispersed) looks slightly white due to light scattering on the rough surface, which reduces the display contrast of the display apparatus. Also, the conventional anti-glare film is problematic in that the rough surface of the anti-glare layer functions as a lens, which causes glare on the display. Furthermore, this conventional anti-glare film is problematic in that back scattering (light generated due to light emitted from the display apparatus being scattered by the resin particles) reduces the display contrast.

As a technique for solving these problems, Patent Document 2 proposes a anti-glare member having a anti-glare layer made of black fine particles and a transparent binder.

However, in the anti-glare member of Patent Document 2, a black pigment is dispersed through the entire black resin fine particles used, that is, through all portions including inside of the particles, and thus, the light transmission is extremely low. Accordingly, there is a problem in which the display of the display apparatus using this anti-glare member becomes dark.

The present invention was made in view of these circumstances, and it is an object thereof to provide colored resin particles having excellent adhesiveness, a method for producing the same, and resin compositions, molded bodies, light-diffusing plates, coating materials, coated products, optical films, anti-glare films, and external preparations.

In order to achieve the above-described object, the present invention is directed to colored resin particles (1) surface-coated with a polymer derived from a nitrogen-containing aromatic compound, wherein each of the particles has an irregular shape with a circular appearance when viewed from a direction in which a projected area thereof becomes maximum, and with a non-circular appearance when viewed from a direction in which the projected area thereof becomes minimum, wherein:
the nitrogen-containing aromatic compound is pyrrole or a pyrrole derivative; the resin particles have any one of a recessed shape, a hemispherical shape, a mushroom-like shape, or a double convex lens-like shape; and
the resin particles having the double convex lens-like shape are obtainable by allowing seed particles to absorb a polymerizable vinyl-based monomer in an aqueous emulsion, the polymerizable vinyl-based monomer containing 5 to 50% by weight of a polyfunctional monomer in which one molecule has two or more polymerizable vinyl groups, and by polymerizing the absorbed polymerizable vinyl-based monomer.

Since each of the colored resin particles (1) has an irregular shape with a circular appearance when viewed from a direction in which a projected area thereof becomes maximum, and with a non-circular appearance when viewed from a direction in which the projected area thereof becomes minimum, these particles have larger rolling friction and better adhesiveness than those of spherical colored resin particles. Accordingly, for example, if the colored resin particles (1) are used in a cosmetic material such as make-up cosmetic material compositions (e.g., mascara) or hair dyeing compositions, a cosmetic material that has excellent adhesiveness when being adhered to the eyelashes, the eyebrows, the skin, or the hair and that does not easily come off can be obtained.

Furthermore, since each of the colored resin particles (1) has a surface on which a coating layer of a polymer derived from a nitrogen-containing aromatic compound is formed, these particles are colored while ensuring light transmission to some extent. Accordingly, the colored resin particles (1) can be used to form a anti-glare member such as a anti-glare film. If the colored resin particles (1) are used to form a anti-glare member, an increase in back scattering light from a display apparatus, which is a light source, can be suppressed, so that display contrast reduction and glare are suppressed. Furthermore, specular reflection of external light on a display face on which the anti-glare member is disposed can be prevented.

With the colored resin particles (1), the coating layer of a polymer derived from a nitrogen-containing aromatic compound can be uniformly and evenly formed on the surfaces of the resin particles. Furthermore, black colored resin particles can be provided. The black colored resin particles are preferable for a cosmetic material such as make-up cosmetic material compositions (e.g., mascara) or hair dyeing compositions, and preferable also for the above-described anti-glare member.

Furthermore, the present invention is preferably directed to colored resin particles (2) according to the colored resin particles (1), wherein the resin particles are obtained by polymerizing a polymerizable vinyl-based monomer containing (meth)acrylic acid ester having an alkylene oxide group.

With the colored resin particles (2), colored resin particles having desired irregular shapes, in particular, colored resin particles having a hemispherical shape or a shape with a horseshoe cross-section can be easily realized.

Moreover, the present invention is directed to a method (1) for producing colored resin particles, including: a step of obtaining resin particles each of which has an irregular shape with a circular appearance when viewed from a direction in which a projected area thereof becomes maximum, and with a non-circular appearance when viewed from a direction in which the projected area thereof becomes minimum, by polymerizing a polymerizable vinyl-based monomer; and a step of coating the surfaces of the obtained resin particles with a polymer derived from a nitrogen-containing aromatic compound, wherein:
the nitrogen-containing aromatic compound is pyrrole or a pyrrole derivative; the resin particles have any one of a recessed shape, a hemispherical shape, a mushroom-like shape, or a double convex lens-like shape; and
the step of obtaining resin particles having the double convex lens-like shape comprises allowing seed particles to absorb a polymerizable vinyl-based monomer in an aqueous emulsion, the polymerizable vinyl-based monomer containing 5 to 50% by weight of a polyfunctional monomer in which one molecule has two or more polymerizable vinyl groups, and polymerizing the absorbed polymerizable vinyl-based monomer.

According to the production method (1), the colored resin particles (1) can be produced. Accordingly, colored resin particles having excellent adhesiveness can be produced.

Furthermore, the present invention is preferably directed to a method (2) for producing colored resin particles according to the production method (1), wherein the polymerizable vinyl-based monomer contains (meth)acrylic acid ester having an alkylene oxide group.

According to the production method (1), colored resin particles controlled to have any irregular shape and any size can be easily produced.

Furthermore, the present invention is preferably directed to a method (3) for producing colored resin particles according to the production method (1) or (2), wherein the step of coating with a polymer derived from a nitrogen-containing aromatic compound includes dispersing the resin particles in an aqueous medium containing 0.5 to 2.0 molar equivalents of alkali metal salt of inorganic peracid with respect to the nitrogen-containing aromatic compound, thereby forming a dispersion, adding the nitrogen-containing aromatic compound to the dispersion, and stirring the resulting mixture, so as to coat the surfaces of the resin particles with the polymer of the nitrogen-containing aromatic compound.

According to the production method (3), colored resin particles can be produced in which the coating layer of a polymer of a nitrogen-containing aromatic compound is uniformly and evenly formed on the entire surfaces of the resin particles.

Moreover, the present invention is directed to a resin composition, containing the colored resin particles of the present invention.

Since the resin composition of the present invention contains the colored resin particles of the present invention, it can be used to form a colored molded body having light-diffusing properties. Furthermore, with the resin composition of the present invention, a molded body having higher transparency than that of a molded body using spherical colored resin particles can be obtained without impairing the physical properties required for the molded body.

Moreover, the present invention is directed to a molded body, obtained by molding the resin composition of the present invention.

Accordingly, a colored molded body having light-diffusing properties can be provided. Furthermore, a molded body having higher transparency than that of a molded body using spherical colored resin particles can be provided without impairing the physical properties required for the molded body.

Moreover, the present invention is directed to a light-diffusing plate, containing the colored resin particles of the present invention.

Accordingly, a colored light-diffusing plate can be provided. Furthermore, a light-diffusing plate having higher transparency than that of a light-diffusing plate using spherical colored resin particles can be obtained without impairing the physical properties required for the light-diffusing plate.

Moreover, the present invention is directed to a coating material, containing the colored resin particles of the present invention.

Since the coating material of the present invention contains the colored resin particles of the present invention, it can be used to form a colored coating film having light-diffusing properties. Furthermore, with the coating material of the present invention, a coating film having higher transparency than that of a coating film using spherical colored resin particles can be obtained without impairing the physical properties required for the coating film.

Moreover, the present invention is directed to a coated product, obtained by applying the coating material of the present invention to a base.

Accordingly, a colored coated product having light-diffusing properties can be provided. Furthermore, a coated product having higher transparency than that of a coated product using spherical colored resin particles can be obtained without impairing the physical properties required for the coated product.

Moreover, the present invention is directed to an optical film and a anti-glare film, obtained by applying the coating material of the present invention to a transparent base film.

Since the colored resin particles of the present invention have been surface-coated with a polymer derived from a nitrogen-containing aromatic compound, they have light transmission to some extent, and, at the same time, have a function of preventing light scattering as in known black resin particles in which a black pigment is dispersed through all portions including inside of the particles. Accordingly, if the optical film and the anti-glare film of the present invention are arranged on the surface of a display apparatus, they can suppress display contrast reduction and glare, while sufficiently maintaining the brightness of display by the display apparatus, and while sufficiently maintaining the effect of preventing specular reflection of external light on the display face. Furthermore, the colored resin particles of the present invention can suppress display contrast reduction and glare, compared with colored resin particles made of spherical resin particles and surface-coated with a polymer derived from a nitrogen-containing aromatic compound.

Furthermore, since the nitrogen-containing aromatic compound in the optical film and the anti-glare film of the present invention is pyrrole or a pyrrole derivative, the above-described effect can be made more significant. Furthermore, if the colored resin particles in the optical film and the anti-glare film of the present invention have a sphere-equivalent volume mean particle diameter of 1 to 10 µm, these films can be more preferably used as a anti-glare member.

Moreover, the present invention is directed to an external preparation, containing the colored resin particles.

Since each of the colored resin particles has an irregular shape, these particles have larger rolling friction and better adhesiveness than those of spherical colored resin particles. Accordingly, an external preparation using the colored resin particles, examples of which include a cosmetic material such as make-up cosmetic material compositions (e.g., mascara) or hair dyeing compositions, has excellent adhesiveness when being adhered to the eyelashes, the eyebrows, the skin, or the hair, and does not easily come off.

The present invention can provide colored resin particles having excellent adhesiveness, a method for producing the same, and resin compositions, molded bodies, light-diffusing plates, coating materials, coated products, optical films, anti-glare films, and external preparations using the colored resin particles.
FIG. 1A shows schematic explanatory views of an irregular particle with a recessed cross-section, having one cut-out portion radially extending therethrough, according to the present invention.
FIG. 1B shows schematic explanatory views of a mushroom-like irregular particle according to the present invention.
FIG. 1C shows schematic explanatory views of a hemispherical irregular particle according to the present invention.
FIG. 1D shows schematic explanatory views of a double convex lens-like irregular particle according to the present invention.
FIG. 2 is a transmission electron micrograph showing a hemispherical colored resin particle obtained in Example 1 of the present invention.
FIG. 3 is a scanning electron micrograph showing hemispherical colored resin particles obtained in Example 1 of the present invention.
FIG. 4 is a scanning electron micrograph showing double convex lens-like colored resin particles obtained in Example 2 of the present invention.
FIG. 5 is a transmission electron micrograph showing a double convex lens-like colored resin particle obtained in Example 2 of the present invention.
FIG. 6 is a scanning electron micrograph showing colored resin particles with a recessed cross-section, having one cut-out portion radially extending therethrough, obtained in Example 3 of the present invention.
FIG. 7 is a transmission electron micrograph showing a colored resin particle with a recessed cross-section, having one cut-out portion radially extending therethrough, obtained in Example 3 of the present invention.
FIG. 8 is a scanning electron micrograph showing mushroom-like colored resin particles obtained in Example 4 of the present invention.
FIG. 9 is a transmission electron micrograph showing a mushroom-like colored resin particle obtained in Example 4 of the present invention.

Hereinafter, colored resin particles and a method for producing the same according to an embodiment of the present invention will be described.

Colored resin particles according to present invention are colored resin particles surface-coated with a polymer derived from a nitrogen-containing aromatic compound, wherein each of the particles has an irregular shape with a circular appearance when viewed from a direction in which a projected area thereof becomes maximum, and with a non-circular appearance when viewed from a direction in which the projected area thereof becomes minimum. Generally, the resin particles are preferably obtained by polymerizing a polymerizable vinyl-based monomer.

Furthermore, a method for producing colored resin particles according to an embodiment includes a step of obtaining resin particles each of which has an irregular shape, by polymerizing a polymerizable vinyl-based monomer, and a step of coating the surfaces of the obtained resin particles with a polymer derived from a nitrogen-containing aromatic compound.

### Irregular Shape

The colored resin particles, and the resin particles used to produce the same of the present invention are particles each of which has an irregular shape (hereinafter, referred to as irregular particles) with a circular appearance when viewed from a direction in which a projected area thereof becomes maximum, and with a non-circular appearance when viewed from a direction in which the projected area thereof becomes minimum. The irregular shape is any one of a recessed shape, a hemispherical shape, a double convex lens-like shape, and a mushroom-like shape. Examples of the recessed shape include a shape with a recessed cross-section, having one cut-out portion radially extending therethrough (hereinafter, also referred to as a shape with a horseshoe cross-section), and a concave lens-like shape not with a horseshoe cross-section. The irregular shape is particularly preferably any one of a shape with a horseshoe cross-section, a hemispherical shape, a double convex lens-like shape, and a mushroom-like shape. Furthermore, the colored resin particles of the present invention, and the resin particles used to produce the same preferably have the same irregular shape, and preferably have irregular shapes arranged in an orderly manner.

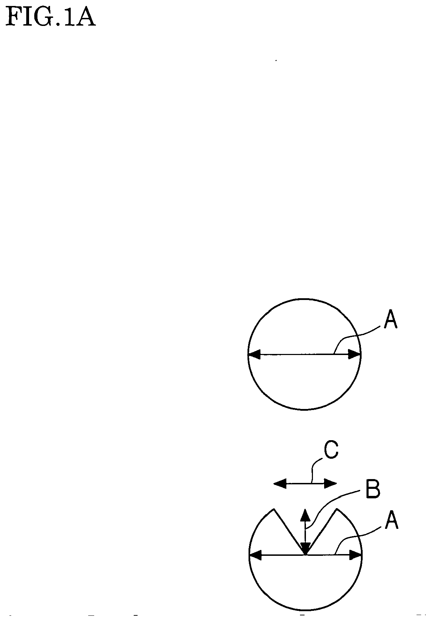

The above-mentioned four types of shapes of the irregular particles will be described with reference to FIGS. 1A to 1D.

FIG. 1A shows projection views of an irregular particle with a horseshoe cross-section, wherein the upper view shows the case in which a projected area thereof becomes maximum, and the lower view shows the case in which the projected area thereof becomes minimum. In the view in which the projected area becomes maximum, the particle has a circular appearance. Furthermore, in the view in which the projected area becomes minimum, the particle has an appearance in the shape obtained by combining a sector and a recess in conformity with the projection view having the cut-out portion. In this example, the irregular particle with a horseshoe cross-section is such that the recess has a depth B that is 0.1 to 0.9 times the particle diameter A of the irregular particle, and has an opening width C that is 0.1 to 0.95 times the particle diameter A.

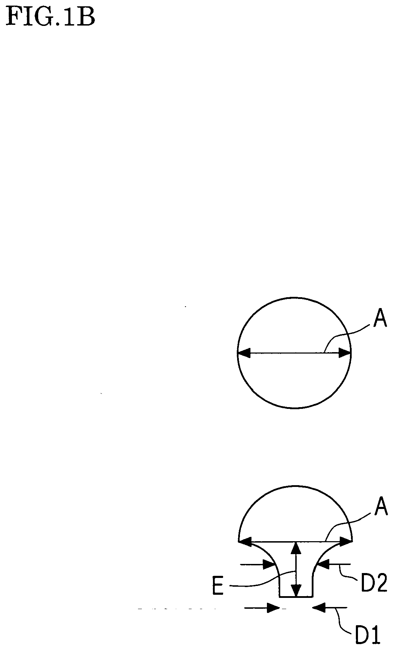

FIG. 1B shows projection views of a mushroom-like irregular particle, wherein the upper view shows the case in which a projected area thereof becomes maximum, and the lower view shows the case in which the projected area thereof becomes minimum. In the view in which the projected area becomes maximum, the particle has a circular appearance. The mushroom-like irregular particle can be considered to include a cap portion and a stem portion, and is such that a bottom width D1 of the stem portion is 0.1 to 0.8 times the particle diameter A of the resin particle, an intermediate width D2 of the stem portion is 0.2 to 0.9 times the particle diameter A of the resin particle, and a height E in the stem length direction is 0.2 to 1.5 times the particle diameter A of the irregular particle.

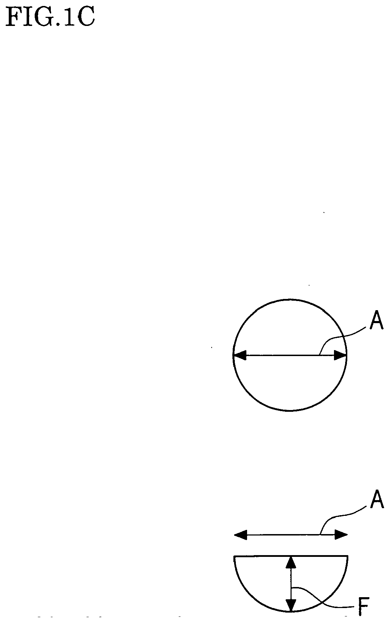

FIG. 1C shows projection views of a hemispherical irregular particle, wherein the upper view shows the case in which a projected area thereof becomes maximum, and the lower view shows the case in which the projected area thereof becomes minimum. In the view in which the projected area becomes maximum, the particle has a circular appearance. In this example, the hemispherical irregular particle is such that, in the drawing showing the hemispherical shape in which the projected area thereof becomes minimum, a height F of the irregular particle is 0.2 to 0.8 times the particle diameter A of the irregular particle.

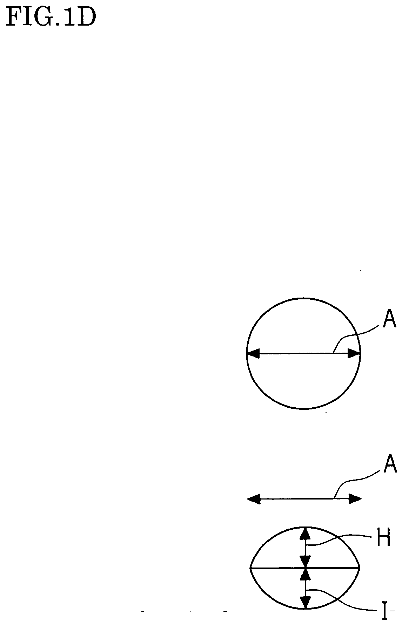

FIG. 1D shows projection views of a double convex lens-like irregular particle (this shape is similar to that of a stone used in a board game called "Go"), wherein the upper view shows a circular shape in which a projected area thereof becomes maximum, and the lower view shows the case in which the projected area thereof becomes minimum. In this example, the double convex lens-like irregular particle is such that, in the lower drawing in which the projected area thereof becomes minimum, each of heights H and I of the convex lenses is 0.2 to 0.8 times the particle diameter A of the irregular particle.

In FIGS. 1A to 1D, the particle diameter A may be set within a range of 0.5 to 30 µm. Furthermore, the sphere-equivalent volume mean particle diameter of the irregular particle may be set within a range of 0.5 to 30 µm.

Note that FIGS. 1A to 1D are views showing shapes for elucidating the shapes of the irregular particles.

The shapes of the irregular particles can be controlled by adjusting, as appropriate, proportions of resin particle starting materials used, polymerization conditions when obtaining resin particles, and the like. For example, the shapes can be controlled by adjusting the composition of seed particles, the weight average molecular weight of seed particles, the amount of polymerizable vinyl-based monomer used with respect to the seed particles, and the amount of (meth)acrylic acid ester having an alkylene oxide group used. For example, if the amount of polymerizable vinyl-based monomer used with respect to the seed particles is increased, irregular particles with a horseshoe cross-section tend to be obtained. Furthermore, if (meth)acrylic acid ester having an alkylene oxide group is not used, double convex lens-like irregular particles tend to be obtained. If a small amount of (meth)acrylic acid ester having an alkylene oxide group is used, hemispherical irregular particles tend to be obtained, and, if the amount of (meth)acrylic acid ester having an alkylene oxide group used is increased, irregular particles with a horseshoe cross-section tend to be obtained. Furthermore, if the molecular weight of the seed particles is low, irregular particles with a horseshoe cross-section tend to be obtained, if the molecular weight of the seed particles is increased, hemispherical irregular particles tend to be obtained, and, if the molecular weight of the seed particles is further increased, mushroom-like irregular particles tend to be obtained.

### Starting Materials for Producing the Resin Particles and Method for Producing the Same

Next, starting materials for producing the resin particles and a method for producing the same will be described. The method for producing the resin particles is preferably a so-called seed polymerization method that allows seed particles to absorb a polymerizable vinyl-based monomer in an aqueous emulsion, and polymerizes the absorbed polymerizable vinyl-based monomer.

### (1) Seed Particles

The seed particles are preferably resin particles derived from a monomer containing (meth)acrylic acid ester whose ester portion contains an alkyl group having 3 or more and less than 6 carbon atoms (i.e., a condensate of alkyl alcohol having 3 or more and less than 6 carbon atoms and (meth)acrylic acid). The monomer for forming the seed particles preferably contains 50% by weight or more of (meth)acrylic acid ester whose ester portion contains an alkyl group having 3 or more and less than 6 carbon atoms. The resin particles derived from such a monomer tend to be non-spherical (irregular) particles. Examples of the alkyl group include linear alkyl groups such as *n*-propyl, *n*-butyl; and *n*-pentyl, and branched alkyl groups such as isopropyl, isobutyl, and *t*-butyl. Specific examples of the (meth)acrylic acid ester include monomers such as *n*-propyl (meth)acrylate, isopropyl (meth)acrylate, *n*-butyl (meth)acrylate, isobutyl (meth)acrylate, and *t*-butyl (meth)acrylate. These monomers may be used alone or in a combination of two or more. Of these, (meth)acrylic acid ester having a branched alkyl group (e.g., isopropyl, isobutyl, and *t*-butyl) is preferable because non-spherical (irregular) resin particles are easily obtained.

Note that, in this specification, "(meth)acrylic" refers to acrylic or methacrylic, and "(meth)acrylate" refers to acrylate or methacrylate.

The weight average molecular weight of the seed particles is preferably in a range of 150000 to 1000000, more preferably in a range of 200000 to 800000, as defined by gel permeation chromatography (GPC). If the weight average molecular weight is 1000000 or less, irregular resin particles are easily obtained, and spherical particles can be prevented from being mixed therein. If the weight average molecular weight is set at 1000000 or less, the ability of the seed particles to absorb a monomer can be prevented from being lowered, and thus, a situation can be prevented in which the monomer is not absorbed and is independently polymerized. As a result, spherical resin particles, which are different from the target resin particles, can be prevented from being produced. On the other hand, if the weight average molecular weight of the seed particles is set at 150000 or more, phase separation between the seed particles and the polymerizable vinyl-based monomer is performed as appropriate, and thus, a situation can be prevented in which the target irregular resin particles are hardly obtained.

Note that there is no particular limitation on the size and the shape of the seed particles. As the seed particles, typically, spherical particles having a particle diameter of 0.1 to 5 µm are used.

### (2) Method for Producing the Seed Particles

There is no particular limitation on the method for producing the seed particles, and examples thereof include known methods such as emulsion polymerization, soap-free emulsion polymerization, seed polymerization, and suspension polymerization. The production method is preferably emulsion polymerization, soap-free emulsion polymerization, or seed polymerization method, in view of the uniformity of the particle diameter of the seed particles and the simplicity of the production method.

The monomer for producing the seed particles may be polymerized in the presence of a molecular weight control agent. Examples of the molecular weight control agent include chain transfer agent such as: *α*-methylstyrene dimmer; mercaptans such as *n*-octyl mercaptan and *t*-dodecyl mercaptan; terpenes such as γ-terpinene and dipentene; and halogenated hydrocarbons such as chloroform and carbon tetrachloride. The molecular weight control agent is preferably used in a range of 0.1 to 10 parts by weight with respect to 100 parts by weight of monomer for producing the seed particles.

### (3) Polymerizable Vinyl-based Monomer

The polymerizable vinyl-based monomer is preferably a monomer containing 5 to 50% by weight of cross-linkable monomer. There is no particular limitation on the cross-linkable monomer, and any known cross-linkable monomer may be used. Examples of the cross-linkable monomer include polyfunctional monomers in which one molecule has two or more polymerizable vinyl groups (polymerizable vinyl groups or substituted vinyl groups), such as ethylene glycol dimethacrylate, trimethylolpropane trimethacrylate, and divinylbenzene. The amount of cross-linkable monomer used is 5 to 50% by weight with respect to the total amount of polymerizable vinyl monomer. If the percentage of cross-linkable monomer is less than 5% by weight or more than 50% by weight, the degree of irregularity of particles may be lowered, and particles close to spherical may be obtained. The use amount is more preferably 10 to 40% by weight.

As necessary, the polymerizable vinyl-based monomer may contain monomers other than the cross-linkable monomer. Examples of other monomers include: (meth)acrylic acid; (meth)acrylic acid derivatives such as methyl (meth)acrylate, ethyl (meth)acrylate, *n*-butyl (meth)acrylate, isobutyl (meth)acrylate, *t*-butyl (meth)acrylate, (meth)acrylamide, 2-hydroxylethyl (meth)acrylate, and glycidyl (meth)acrylate; vinyl acetate; and acrylonitrile.

Of these, (meth)acrylic acid ester having an alkylene oxide group is more preferable as other monomers because non-spherical (irregular) particles are easily obtained. Examples of the (meth)acrylic acid ester include a compound represented by General Formula below.

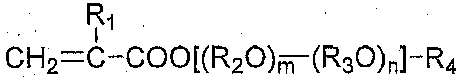

In General Formula above, R₁ represents H or CH₃, R₂ and R₃ are different alkylene groups selected from C₂H₄, C₃H₆, C₄H₈, and C₅H₁₀, m is 0 to 50, n is 0 to 50 (where m and n are not simultaneously 0), and R₄ represents H or CH₃.

Note that, in the monomer represented by General Formula above, if m is larger than 50 or if n is larger than 50, the polymerization stability may be lowered, and stuck particles may be generated. Here, m and n are preferably in a range of 0 to 30, more preferably in a range of 0 to 15.

As the (meth)acrylic acid ester having an alkylene oxide group, commercially available products may be used. Examples of the commercially available products include Blemmer (registered trademark) series manufactured by NOF Corporation. Furthermore, in the Blemmer (registered trademark) series, examples of suitable products include Blemmer (registered trademark) 50PEP-300 (R₁ represents CH₃, R₂ represents C₂H₅, R₃ represents C₃H₆, this product is a mixture with an average m of 3.5 and an average n of 2.5, and R₄ represents H), Blemmer (registered trademark) 70PEP-350B (R₁ represents CH₃, R₂ represents C₂H₅, R₃ represents C₃H₆, this product is a mixture with an average m of 3.5 and an average n of 2.5, and R₄ represents H), Blemmer (registered trademark) PP-1000 (R₁ represents CH₃, R₃ represents C₃H₆, m is 0, this product is a mixture with an average n of 4 to 6, and R₄ represents H), and Blemmer (registered trademark) PME-400 (R₁ represents CH₃, R₂ represents C₂H₅, this product is a mixture with an average m of 9, n is 0, and R₄ represents CH₃).

The amount of (meth)acrylic acid ester having an alkylene oxide group used is preferably 0 to 40% by weight, more preferably 1 to 40% by weight, even more preferably 5 to 30% by weight, and particularly preferably 10 to 20% by weight, with respect to the total amount of polymerizable vinyl-based monomer. If the amount of (meth)acrylic acid ester having an alkylene oxide group used is more than 40% by weight with respect to the total amount of polymerizable vinyl-based monomer, the polymerization stability may be lowered, and a large amount of stuck particles may be generated.

### (4) Method for Producing the Resin Particles

Hereinafter, a method for producing the resin particles using the seed polymerization method will be described as an example, but the method for producing the resin particles is not limited to this method.

In the method for producing the resin particles using the seed polymerization method, first, the seed particles are added to an aqueous emulsion made of the polymerizable vinyl-based monomer and an aqueous medium. Examples of the aqueous medium include water, and a mixture medium of water and water-soluble solvent (e.g., lower alcohol having 5 or less carbon atoms).

The aqueous medium preferably contains a surfactant. Examples of the surfactant include all of anionic, cationic, nonionic, and amphoteric surfactants.

Examples of the anionic surfactant include sodium oleate, fatty acid soap such as potassium soap of castor oil, alkyl sulfate salt such as sodium lauryl sulfate and ammonium lauryl sulfate, alkylbenzenesulfonate such as sodium dodecylbenzenesulfonate, alkylnaphthalenesulfonate, alkanesulfonate, dialkyl sulfosuccinate such as sodium dioctyl sulfosuccinate, alkenyl succinate (dipotassium salt), alkyl phosphate ester salt, naphthalenesulfonate formaldehyde condensate, polyoxyethylene alkyl ether sulfate such as polyoxyethylene alkylphenyl ether sulfate and sodium polyoxyethylene lauryl ether sulfate, and polyoxyethylene alkyl sulfate.

Examples of the cationic surfactant include alkylamine salt such as lauryl amine acetate and stearyl amine acetate, and quaternary ammonium salt such as lauryl trimethylammonium chloride.

Examples of the amphoteric surfactant include lauryl dimethylamine oxide, phosphate ester surfactants, and phosphite ester surfactants.

The surfactants described above may be used alone or in a combination of two or more. Of these surfactants, an anionic surfactant is preferable in view of the dispersion stability during polymerization.

The aqueous emulsion can be produced using a known method. For example, the aqueous emulsion can be obtained by adding a polymerizable vinyl-based monomer to an aqueous medium, and dispersing the monomer using a microemulsifier such as a homogenizer, an ultrasonic treatment machine, or a nanomizer. The polymerizable vinyl-based monomer may contain a polymerization initiator as necessary. The polymerization initiator may be mixed in advance in the polymerizable vinyl-based monomer, after which the mixture may be dispersed in an aqueous medium. Alternatively, the polymerization initiator and the polymerizable vinyl-based monomer may be separately dispersed in aqueous media, after which the resultant dispersions may be mixed. The particle diameter of droplets of the polymerizable vinyl-based monomer in the aqueous emulsion is preferably smaller than that of the seed particles because the polymerizable vinyl-based monomer is efficiently absorbed by the seed particles.

The seed particles may be directly added to the aqueous emulsion, or may be added to the aqueous emulsion in a state where the seed particles have been dispersed in an aqueous dispersion medium.

After the seed particles are added to the aqueous emulsion, the seed particles are allowed to absorb the polymerizable vinyl-based monomer. This absorption is performed typically by stirring the aqueous emulsion to which the seed particles have been added, at room temperature (about 20°C) for 1 to 12 hours. Furthermore, the absorption may be facilitated by heating the aqueous emulsion at approximately 30 to 50°C.

The seed particles are swollen by absorbing the polymerizable vinyl-based monomer. The proportion of the polymerizable vinyl-based monomer and the seed particles mixed is such that the polymerizable vinyl-based monomer is contained preferably in a range of 5 to 150 parts by weight, more preferably in a range of 10 to 120 parts by weight, with respect to 1 part by weight of seed particles. If the proportion of the polymerizable vinyl-based monomer mixed with respect to the seed particles becomes too small, an increase in the particle diameter due to polymerization is reduced, and thus, the productivity may be lowered. If the proportion of the polymerizable vinyl-based monomer mixed with respect to the seed particles becomes too large, the polymerizable vinyl-based monomer is not completely absorbed by the seed particles and is independently polymerized through suspension polymerization in the aqueous medium, and thus, abnormal particles may be produced. Note that the end of the absorption may be determined by checking an increase in the particle diameter with observation using an optical microscope.

A polymerization initiator may be added to the aqueous emulsion as necessary. Examples of the polymerization initiator include organic peroxides such as benzoyl peroxide, lauroyl peroxide, ortho-chlorobenzoyl peroxide, ortho-methoxybenzoyl peroxide, 3,5,5-trimethylhexanoyl peroxide, *t*-butyl peroxy-2-ethyl hexanoate, and di-*t*-butyl peroxide, and azo-based compounds such as 2,2'-azobisisobutyronitrile, 1,1'-azobiscyclohexanecarbonitrile, and 2,2'-azobis(2,4-dimethylvaleronitrile). The polymerization initiator is preferably used in a range of 0.1 to 3 parts by weight with respect to 100 parts by weight of polymerizable vinyl-based monomer.

Next, irregular resin particles are obtained by polymerizing the polymerizable vinyl-based monomer that has been absorbed by the seed particles. The polymerization temperature is selected as appropriate according to the types of polymerizable vinyl-based monomer and polymerization initiator. The polymerization temperature is preferably in a range of 25 to 110°C, more preferably in a range of 50 to 100°C. The polymerization reaction is preferably performed at an elevated temperature after the monomer and the polymerization initiator are completely absorbed by the seed particles. After the polymerization is completed, as necessary, the irregular resin particles are centrifuged to remove the aqueous medium, washed with water and solvent, dried, and isolated.

In this polymerization step, in order to improve the dispersion stability of the irregular resin particles, a macromolecular dispersion stabilizer may be added. Examples of the macromolecular dispersion stabilizer include polyvinyl alcohol, polycarboxylic acid, celluloses (hydroxyethylcellulose, carboxymethylcellulose, etc.), and polyvinylpyrrolidone. Furthermore, these macromolecular dispersion stabilizers and an inorganic water-soluble high molecular compound such as sodium tripolyphosphate may be used together. Of these, polyvinyl alcohol and polyvinylpyrrolidone are preferable as the macromolecular dispersion stabilizer. The amount of macromolecular dispersion stabilizer added is preferably 1 to 10 parts by weight with respect to 100 parts by weight of polymerizable vinyl-based monomer.

Furthermore, in order to suppress generation of emulsion particles in the aqueous system, a water-soluble polymerization inhibitor such as nitrites, sulfites, hydroquinones, ascorbic acids, water-soluble vitamin Bs, citric acid, or polyphenols may be used.

Next, a step of producing colored resin particles by surface-coating the irregular resin particles obtained through the above-described steps with a polymer derived from a nitrogen-containing aromatic compound will be described.

### Method for Producing Colored Resin Particles

In the method for producing colored resin particles of the present invention, the irregular resin particles obtained using the above-described seed polymerization method or the like are surface-coated with a polymer derived from a nitrogen-containing aromatic compound. As the method for performing coating with a polymer derived from a nitrogen-containing aromatic compound, a method is preferable in which the resin particles are dispersed in an aqueous medium containing an oxidizer to form a dispersion (an emulsion or a suspension), a nitrogen-containing aromatic compound is added to the dispersion, and the mixture is stirred, so that the resin particles are surface-coated with a polymer of the nitrogen-containing aromatic compound through oxidation polymerization.

### (1) Nitrogen-Containing Aromatic Compound

The nitrogen-containing aromatic compound includes pyrrole and derivatives thereof such as alkyl-substituted compounds (e.g., compounds substituted with an alkyl group having 1 to 4 carbon atoms such as methyl group, ethyl group, propyl group, or butyl group), halogen-substituted compounds (e.g., compounds substituted with a halogen group such as a fluoro group, a chloro group, or a bromo group), and nitrile-substituted compounds. These monomers may be used alone to produce a homopolymer, or in a combination of two or more to produce a copolymer. As the nitrogen-containing aromatic compound, polymers of pyrrole or a pyrrole derivative are preferable because a more uniform coating layer is easily obtained, and black colored resin particles are obtained.

The amount of nitrogen-containing aromatic compound added may be set according to a desired degree of coloring and the like. This amount is in a range of preferably 1 to 30 parts by weight, more preferably 3 to 20 parts by weight, with respect to 100 parts by weight of resin particles. If the amount of nitrogen-containing aromatic compound added is 1 part by weight or more with respect to 100 parts by weight of resin particles, the entire surfaces of the resin particles are uniformly coated with a polymer derived from the nitrogen-containing aromatic compound, and thus, a desired degree of coloring can be obtained. On the other hand, if the amount of nitrogen-containing aromatic compound added is 30 parts by weight or less with respect to 100 parts by weight of resin particles, the added nitrogen-containing aromatic compound can be prevented from being independently polymerized to form products other than the target colored resin particles.

### (2) Oxidizer

Examples of the oxidizer include inorganic acids such as hydrochloric acid, sulfuric acid, and chlorosulfonic acid, organic acids such as alkylbenzenesulfonic acid and alkylnaphthalenesulfonic acid, metal halides such as ferric chloride and aluminum chloride, halogen acids such as potassium perchlorate, and peracids such as potassium persulfate, ammonium persulfate, sodium persulfate, and hydrogen peroxide. These oxidizers may be used alone or in a combination. The oxidizer is preferably an alkali metal salt of inorganic peracid. Specific examples of the alkali metal salt of inorganic peracid include potassium persulfate and sodium persulfate.

The amount of oxidizer used is preferably 0.5 to 2.0 molar equivalents with respect to the total amount of nitrogen-containing aromatic compound. If the amount of oxidizer used is 0.5 molar equivalents or more with respect to the total amount of nitrogen-containing aromatic compound, the entire surfaces of the resin particles are uniformly coated with a coating layer containing a polymer of the nitrogen-containing aromatic compound, and a desired degree of coloring can be obtained. On the other hand, if the amount of oxidizer used is 2.0 molar equivalents or less with respect to the total amount of nitrogen-containing aromatic compound, the added nitrogen-containing aromatic compound can be prevented from being independently polymerized to form products other than the target colored resin particles.

### (3) Aqueous Medium

There is no particular limitation on the aqueous medium to which the oxidizer is added, as long as the nitrogen-containing aromatic compound can be dissolved or dispersed therein. Examples of the aqueous medium include water, and a mixture medium of water and alcohols such as methanol, ethanol, *n*-propanol, isopropanol, *n*-butanol, or *t*-butanol, ethers such as diethyl ether, isopropyl ether, butyl ether, methyl cellosolve, or tetrahydrofuran, or ketones such as acetone, methyl ethyl ketone, or diethyl ketone.

The aqueous medium to which the oxidizer has been added preferably has a pH of 3 or more. If the pH is 3 or more, the entire surfaces of the resin particles are uniformly coated with a coating layer containing a polymer of the nitrogen-containing aromatic compound, and a desired degree of coloring can be obtained. In order to stably perform coating, the pH is more preferably adjusted in a range of 3 to 10.

### (4) Surfactant

Furthermore, a surfactant may be added to the aqueous medium. Examples of the surfactant include all of an anionic surfactant, a cationic surfactant, an amphoteric surfactant, and a nonionic surfactant.

Examples of the anionic surfactant include sodium oleate, fatty acid soap such as potassium soap of castor oil, alkyl sulfate salt such as sodium lauryl sulfate and ammonium lauryl sulfate, alkylbenzenesulfonate such as sodium dodecylbenzenesulfonate, alkylsulfonate, alkylnaphthalenesulfonate, alkanesulfonate, sulfosuccinate, dialkyl sulfosuccinate, alkyl phosphate ester salt, naphthalenesulfonate formaldehyde condensate, polyoxyethylene alkylphenyl ether sulfate, and polyoxyethylene alkyl sulfate.

Examples of the nonionic surfactant include polyoxyethylene alkyl ether, polyoxyethylene alkylphenyl ether, polyoxyethylene fatty acid ester, sorbitan fatty acid ester, polyoxysorbitan fatty acid ester, polyoxyethylene alkylamine, glycerin fatty acid ester, and oxyethylene-oxypropylene block polymer.

Examples of the cationic surfactant include alkylamine salt such as lauryl amine acetate and stearyl amine acetate, and quaternary ammonium salt such as lauryl trimethylammonium chloride.

Examples of the amphoteric surfactant include lauryl dimethylamine oxide, phosphate ester surfactants, and phosphite ester surfactants. The surfactants described above may be used alone or in a combination of two or more. The amount of surfactant added is preferably in a range of 0.0001 to 1 part by weight with respect to 100 parts by weight of aqueous medium.

Furthermore, other than the surfactant, a macromolecular dispersion stabilizer may be added to the aqueous medium. Examples of the macromolecular dispersion stabilizer include polyacrylic acid, copolymers thereof, and neutralized products thereof, polymethacrylic acid, copolymers thereof, and neutralized products thereof, polyvinylpyrrolidone, and hydroxypropylcellulose (HPC). The macromolecular dispersion stabilizer may be used together with the above-described surfactant.

### (5) Oxidation Polymerization

In the method for producing colored resin particles using oxidation polymerization, the resin particles are dispersed in an aqueous medium containing an oxidizer to form a dispersion, a nitrogen-containing aromatic compound is added to the dispersion, and the mixture is subjected to oxidation polymerization with stirring, so that colored resin particles having an irregular shape and surface-coated with a polymer derived from the nitrogen-containing aromatic compound are obtained. The oxidation polymerization temperature is preferably in a range of -20 to 40°C, and the oxidation polymerization duration is preferably in a range of 0.5 to 10 hours.

Note that, as necessary, the emulsion in which the colored resin particles are dispersed is centrifuged to remove the aqueous medium, washed with water and solvent, dried, and isolated.

As a method for coating resin particles with a polymer, the foregoing embodiment described a method in which irregular resin particles obtained using the seed polymerization method or the like are mixed with the nitrogen-containing aromatic compound in an aqueous medium containing an alkali metal salt of inorganic peracid, and the mixture is subjected to oxidation polymerization. However, the method for coating resin particles with a polymer is not limited to this method, and, for example, a method may be also employed in which the nitrogen-containing aromatic compound is polymerized in advance, and the obtained polymer is applied to coat the resin particles using a dry method. Examples of the dry method include a method using a ball mill, a method using a V-type mixer, a method using a high-speed fluidized bed dryer, a method using a hybridizer, and a mechanofusion method.

### Colored Resin Particles

The colored resin particles are preferably such that the entire surfaces of the resin particles are uniformly coated with a coating layer containing a polymer derived from the nitrogen-containing aromatic compound. In this example, "uniform" refers to a state in which the deflection of the thickness of the coating layer containing a polymer derived from the nitrogen-containing aromatic compound coating the resin particles is low. The deflection is preferably 50% or less, more preferably 40% or less.

The thickness of the coating layer is preferably in a range of 30 to 300 nm, more preferably in a range of 50 to 200 nm. If the thickness of the coating layer is 30 nm or more, a sufficient degree of coloring can be obtained, and desired characteristics (e.g., anti-glare properties, etc.) can be obtained. On the other hand, if the thickness of the coating layer is 300 nm or less, a decrease in the light transmission can be suppressed. For example, the display of a display apparatus provided with a anti-glare member using these resin particles can be prevented from becoming dark.

There is no particular limitation on the sphere-equivalent volume mean particle diameter of the colored resin particles. In view of use for a cosmetic material composition, this particle diameter is preferably 1 to 50 µm, more preferably 1 to 30 µm. If the sphere-equivalent volume mean particle diameter of the colored resin particles is 1 µm or more, the effect of improving adhesiveness when being used as a cosmetic material can be obtained. On the other hand, if the sphere-equivalent volume mean particle diameter of the colored resin particles is 50 µm or less, a cosmetic material that does not impair application properties or touch feeling can be obtained.

Furthermore, in view of use for a anti-glare member, the sphere-equivalent volume mean particle diameter of the colored resin particles is preferably 1 to 10 µm, more preferably 3 to 7 µm. If the sphere-equivalent volume mean particle diameter of the colored resin particles is 1 µm or more, fine roughness is reliably formed on the anti-glare member, and desired anti-glare properties can be obtained. On the other hand, if the sphere-equivalent volume mean particle diameter of the colored resin particles is 10 µm or less, the fine roughness can be easily adjusted, and desired anti-glare properties can be obtained through this adjustment.

Furthermore, the coefficient of variation (CV) in the particle diameter of the colored resin particles is preferably 30% or less, more preferably 20% or less, in order to obtain a coating film that has improved anti-glare properties, can prevent glare, and is uniform.

### Use of the Colored Resin Particles

Each of the colored resin particles according to the present invention has an irregular shape with a circular appearance when viewed from a direction in which a projected area thereof becomes maximum, and with a non-circular appearance when viewed from a direction in which the projected area thereof becomes minimum, and is surface-coated with a coating layer made of a polymer derived from a nitrogen-containing aromatic compound. Accordingly, colored resin particles can be obtained that have the degree of coloring (e.g., black) resulting from this polymer and that can be applied to various types of use typified by cosmetic materials.

For example, the colored resin particles are colored only at their surface portion, and thus, they do not significantly reduce the amount of light that is allowed to pass through the particles, compared with known colored particles that are colored through all portions including the internal portion of the particles. Accordingly, a film or a sheet in which these colored resin particles are dispersed can be preferably used in the field of anti-glare members having desired stable total light transmittance, for example. Such a. anti-glare member can be obtained by applying a mixture of the colored resin particles according to the present invention and a binder resin to a transparent- base resin. Furthermore, since each of the colored resin particles has an irregular shape with a circular appearance when viewed from a direction in which a projected area thereof becomes maximum, and with a non-circular appearance when viewed from a direction in which the projected area thereof becomes minimum, the adhesiveness can be improved.

Furthermore, as use other than those described above, the colored resin particles can be preferably used also as an additive to a coating agent (coating composition) for a conductive film, an antistatic film, an electromagnetic shielding material, an information recording paper, and the like, due to the conductivity of the polymer (e.g., polypyrrole, etc.) derived from the nitrogen-containing aromatic compound. Furthermore, these colored resin particles can be preferably used also as an additive to a cosmetic material, a coating material, ink, adhesive, pressure-sensitive adhesive, and the like, due to the coloring properties (e.g., black) of the polymer derived from the nitrogen-containing aromatic compound.

### Resin Composition

The resin composition of the present invention contains the colored resin particles of the present invention. There is no particular limitation on the shape of the colored resin particles used in the resin composition of the present invention, as long as it is any one of a recessed shape, a hemispherical shape, a double convex lens-like shape, and a mushroom-like shape, preferably a double convex lens-like shape.

The resin composition of the present invention more preferably contains a base resin. The resin composition of the present invention further containing a base resin diffuses light, and thus, it can be used as a light-diffusing resin composition for molding a light-diffusing member such as a light-diffusing plate.

Examples of the base resin include: polycarbonate-based resins; vinyl chloride-based resins such as polyvinyl chloride and vinyl chloride-vinylidene chloride copolymer; vinyl ester-based resins such as polyvinyl acetate and vinyl acetate-ethylene copolymer; styrene-based resins such as polystyrene, styrene-acrylonitrile copolymer, styrene-butadiene-acrylonitrile copolymer, styrene-butadiene block copolymer, styrene-isoprene block copolymer, and styrene-methyl methacrylate copolymer; (meth)acrylic acid ester-based resins such as poly(meth)acrylic acid ester, (meth)acrylic acid ester-acrylonitrile copolymer, and (meth)acrylic acid ester-styrene copolymer; polyester-based resins such as condensate of terephthalic acid and ethylene glycol and condensate of adipic acid and ethylene glycol; polyolefin-based resins such as polyethylene, polypropylene, chlorinated polyethylene, chlorinated polypropylene, carboxyl modified polyethylene, polyisobutylene, and polybutadiene; urethane-based resins; epoxy-based resins; silicone-based resins; and fluorine-based resins. These base resins may be used alone or in a combination of two or more.

When intending to obtain a resin composition having excellent transparency, a base resin having a refractive index similar to that of the colored resin particles is preferably selected. When intending to obtain a resin composition having excellent light diffusion, a base resin having a refractive index sufficiently different from that of the colored resin particles is preferably selected. When using the resin composition of the present invention as a light-diffusing resin composition for molding a light-diffusing member such as a light-diffusing plate, a polycarbonate-based resin is preferable as the base resin. Accordingly, the resin composition and a molded body obtained by molding this resin composition can be provided with excellent impact resistance and excellent light transmission.

Examples of the polycarbonate-based resin include aromatic polycarbonate-based resins obtained by reacting an aromatic dihydroxy compound with phosgene or diester carbonate using a fusion method or a solution method.

Examples of the aromatic dihydroxy compound include: bis(hydroxyaryl)alkanes such as 2,2-bis(4-hydroxyphenyl)propane, bis(4-hydroxyphenyl)methane, 1,1-bis(4-hydroxyphenyl)ethane, 2,2-bis(4-hydroxyphenyl)butane, 2,2-bis(4-hydroxyphenyl)octane, 2,2-bis(4-hydroxyphenyl)phenylmethane, 2,2-bis(4-hydroxy-1-methylphenyl)propane, bis(4-hydroxyphenyl)naphthylmethane, 1,1-bis(4-hydroxy-t-butylphenyl)propane, 2,2-bis(4-hydroxy-3-bromophenyl)propane, -2,2-bis(4-hydroxy-3,5-dimethylphenyl)propane, 2,2-bis(4-hydroxy-3-chlorophenyl)propane, 2,2-bis(4-hydroxy-3,5-dichlorophenyl)propane, and 2,2-bis(4-hydroxy-3,5-dibromophenyl)propane; bis(hydroxyaryl)cycloalkanes such as 1,1-bis(4-hydroxyphenyl)cyclopentane, 1,1-bis(4-hydroxyphenyl)cyclohexane, and 1,1-bis(4-hydroxyphenyl)-3,5,5-trimethylcyclohexane; dihydroxyaryl ethers such as 4,4'-dihydroxyphenyl ether and 4,4'-dihydroxy-3,3'-dimethylphenyl ether; dihydroxy diaryl sulfides such as 4,4'-dihydroxy diphenyl sulfide and 4,4'-dihydroxy-3,3'-dimethyl diphenyl sulfide; dihydroxy diaryl sulfoxides such as 4,4'-dihydroxy diphenyl sulfoxide and 4,4'-dihydroxy-3,3'-dimethyl diphenyl sulfoxide; dihydroxy diaryl sulfones such as 4,4'-dihydroxy diphenyl sulfone and 4,4'-dihydroxy-3,3'-dimethyl diphenyl sulfone; and dihydroxy diphenyls such as 4,4'-dihydroxy diphenyl.

These aromatic dihydroxy compounds may be used alone or in a combination of two or more. Furthermore, the aromatic dihydroxy compound may contain any substituent and the like, and may be cross-linked by a cross-linking agent or the like, as long as desired physical properties are not affected. The aromatic dihydroxy compound is preferably 2,2-bis(4-hydroxyphenyl)propane [Bisphenol A] because a resin composition having excellent impact resistance can be obtained. The resin composition of the present invention may contain two or more polycarbonate-based resins.

Typically, the colored resin particles are added preferably in an amount of 0.1 to 30% by weight with respect to the resin composition. If the amount of colored resin particles added is less than 0.1% by weight, it is difficult to obtain desired effects (e.g., light diffusing effect) resulting from the colored resin particles. On the other hand, if the amount of colored resin particles added is more than 30% by weight, the colored resin particles are not uniformly dispersed in the resin composition, which also makes it difficult to obtain desired effects (e.g., light diffusing effect) resulting from the colored resin particles.

The resin composition of the present invention may contain various components such as a fluorescent whitening agent, a thermal stabilizer, or an organic or inorganic ultraviolet absorber. These various components can provide the resin composition with desired physical properties such as fluorescent whitening properties, thermal stability, and ultraviolet absorbing ability.

There is no particular limitation on the fluorescent whitening agent, and examples thereof include an oxazole-based fluorescent whitening agent (e.g., "Kayalight (registered trademark) OS" manufactured by Nippon Kayaku Co., Ltd.), an imidazole-based fluorescent whitening agent, and a rhodamine-based fluorescent whitening agent. If these fluorescent whitening agents are contained in the resin composition in a proportion of preferably 0.0005 to 0.1 parts by weight, more preferably 0.001 to 0.1 parts by weight, and even more preferably 0.001 to 0.05 parts by weight, with respect to 100 parts by weight of base resin, the resin composition can be provided with sufficient fluorescent whitening properties.

There is no particular limitation on the thermal stabilizer, and examples thereof include: organic phosphorous thermal stabilizers such as phosphate ester, phosphite ester, and phosphonate ester; hindered phenolic thermal stabilizers; lactone-based thermal stabilizers; and phosphite-based antioxidants (e.g., "ADK Stab (registered trademark) PEP-36" manufactured by ADEKA Corporation) having a function of suppressing thermal oxidation. If these thermal stabilizers are contained in the resin composition in a proportion of preferably 0.005 to 0.5 parts by weight, more preferably 0.01 to 0.5 parts by weight, and even more preferably 0.001 to 0.3 parts by weight, with respect to 100 parts by weight of base resin, the resin composition can be provided with sufficient thermal stability.

There is no particular limitation on the organic ultraviolet absorber, and examples thereof include: benzophenone-based ultraviolet absorbers such as 2-hydroxy-4-methoxy-5-sulfobenzophenone; benzotriazole-based ultraviolet absorbers such as 2,2'-methylenebis[4-(1,1,3,3-tetramethylbutyl)-6-[2H-benzotriazole-2-yl]pheno 1] ("ADK Stab (registered trademark) LA-31" manufactured by ADEKA Corporation), 2-(2-hydroxy-5-methylphenyl)benzotriazole, 2-(2-hydroxy-5-*t*-butylphenyl)benzotriazole, and 2-(2-hydroxy-3,5-di *t*-butylphenyl)-5-chlorobenzotriazole; hydroxyphenyl triazine-based ultraviolet absorbers; salicylic acid-based ultraviolet absorbers such as *p*-*t*-butylphenyl salicylate and *p*-octylphenyl salicylate; and cyanoacrylate-based ultraviolet absorbers such as 2-ethylhexyl-2-cyano-3,3-diphenylacrylate and ethyl 2-cyano-3,3-diphenylacrylate. If these organic ultraviolet absorbers are contained in the resin composition in a proportion of preferably 0.01 to 2.0 parts by weight, more preferably 0.03 to 2.0 parts by weight, and even more preferably 0.05 to 1.0 parts by weight, with respect to 100 parts by weight of base resin, the resin composition can be provided with sufficient ultraviolet absorbing ability.

Examples of the inorganic ultraviolet absorber include inorganic fine particles (particles having a particle diameter of 0.003 to 0.1 µm, preferably 0.003 to 0.05 µm) such as zinc oxide fine particles, titanium oxide fine particles, cerium oxide fine particles, and zirconium oxide fine particles. If these inorganic ultraviolet absorbers are contained in the resin composition in a proportion of 0.001 to 24% by weight, the resin composition can be provided with sufficient ultraviolet absorbing ability. The inorganic ultraviolet absorbers may be contained in the colored resin particles in a proportion of 1 to 80% by weight. These ultraviolet absorbers may be used alone or in a combination of two or more.

### Molded Body and Light-Diffusing Plate

The molded body of the present invention is obtained by molding the resin composition of the present invention. As the method for molding the resin composition, in view of productivity, a method may be employed in which the resin composition in the form of pellets is molded using a molding process such as injection molding, injection compression molding, or extrusion molding to form a molded body. Also, a method may be employed in which the resin composition is subjected to extrusion molding to form a molded body in the form of a sheet, and this molded body in the form of a sheet is molded using a molding process such as vacuum molding or compressed air molding to form a molded body.

Such a molded body of the present invention diffuses light, and thus, it can be used as a light-diffusing member such as a light-diffusing plate containing colored resin particles. The light-diffusing plate of the present invention contains colored resin particles.

Furthermore, the molded body of the present invention can be used also as electric and electronic components such as illumination covers, car components such as car interior panels, mechanical components, wrapping films for wrapping miscellaneous goods, food, medicine, or the like, containers for food, medicine, or the like, fiber, plates, building structures such as roofs, and the like. Since the colored resin particles contain a polymer derived from pyrrole or a pyrrole derivative, the molded body of the present invention is conductive and antistatic.

Furthermore, as an auxiliary substance for improving weather resistance or processability as necessary, an appropriate amount of plasticizer, stabilizer, antioxidant, flame retardant, antistatic agent, lubricant, macromolecule modifier, and the like may be mixed as necessary into the molded body of the present invention. Furthermore, an extender pigment, a coloring pigment, a dye, and the like may be added to the molded body within a range not impairing the transparency of the molded body.

### Coating Material

The coating material of the present invention contains the colored resin particles of the present invention. There is no particular limitation on the shape of the colored resin particles used in the coating material of the present invention, as long as it is any one of a recessed shape, a hemispherical shape, a double convex lens-like shape, and a mushroom-like shape, preferably a double convex lens-like shape.

The coating material of the present invention preferably contains a resin binder. Examples of the resin binder include various polymers that can form a coating film, such as (meth)acrylic acid ester-based resin (acrylic-based resin), polycarbonate-based resin, polyester-based resin, urethane-based resin, epoxy-based resin, polypropylene-based resin, silicone-based resin, styrene-based resin, fluorine-based resin, cellulose-based resin, and other vinyl-based resins (vinyl chloride-based resin, etc.), modified products thereof, and precursors (monomers, oligomers, or mixtures thereof) of these polymer. These resin binders may be dissolved in solvent or may be present as emulsion in solvent, and are selected as appropriate according to the application purpose.

When intending to obtain a coating film having excellent transparency, a vehicle component having a refractive index similar to that of the colored resin particles is preferably selected. Furthermore, when intending to obtain a coating film having excellent mat properties, a vehicle component having a refractive index sufficiently different from that of the colored resin particles is preferably selected. For example, when combining colored resin particles mainly made of acrylic-based resin and a resin binder made of acrylic-based resin, a coating film having excellent transparency can be obtained, and when combining colored resin particles mainly made of styrene-based resin and a resin binder made of acrylic-based resin, a coating film having excellent mat properties can be obtained.

Examples of the form of the coating material according to the present invention include liquid coating materials in which a solid resin binder is dissolved in solvent and colored resin particles are dispersed in the solvent, liquid coating materials in which a solid resin binder and colored resin particles are dispersed in solvent, powder coating materials in which a solid resin binder and colored resin particles are contained but solvent is not contained, liquid coating materials in which a liquid resin binder is contained but solvent is not contained, and liquid coating materials in which a liquid resin binder is diluted with solvent. Examples of the liquid resin binder include ultraviolet curable or electron beam curable precursors (monomers, oligomers, or mixtures thereof) of polymers.

The solvent used as necessary in the coating material of the present invention may be selected as appropriate according to the characteristics of a coating film that is to be formed from the colored resin particles and the resin binder. Typical examples thereof include water, alcohols (ethyl alcohol, methyl alcohol, isopropyl alcohol, propylene glycol monomethyl ether, etc.), esters (ethyl acetate, isopropyl acetate, butyl acetate, etc.), ketones (acetone, methyl ethyl ketone [MEK], methyl isobutyl ketone), ethers (cellosolve, butyl cellosolve), and terpenes (turpentine, dipentene).

As an auxiliary substance for improving application properties or coating film performance, an appropriate amount of organic or inorganic ultraviolet absorber, dispersant, surfactant, anti-settling agent, humectant, anti-sagging agent, defoaming agent, antioxidant, and the like may be mixed as necessary into the coating material of the present invention. Examples of the compounds that can be used as the ultraviolet absorber and preferable ranges of the amount of the ultraviolet absorber mixed are similar to those in the case of the ultraviolet absorber used as necessary in the resin composition, which was described in the paragraphs for the resin composition. Furthermore, an extender pigment, a coloring pigment, a dye, and the like may be added to the coating material of the present invention within a range not impairing the transparency of the coating film.

The coating material has to be flowable enough to achieve a uniform state, and has to have a solid content with which the resin binder that has been cured is integrated, and thus, the content of colored resin particles in components (components other than the solvent, that is, the solid content if the resin binder is solid) for forming a coating film in the coating material is preferably 95% by weight or less. If the content of colored resin particles in the components for forming a coating film in the coating material is more than 95% by weight, the coating film becomes significantly fragile. Furthermore, the content of colored resin particles in the components for forming a coating film in the coating material is preferably 0.5% by weight or more. Accordingly, the matting effect and the coloring effect of the colored resin particles can be sufficiently obtained.

### Coated Product

The coated product of the present invention is obtained by applying (performing coating with) the coating material of the present invention over a base. That is to say, the coated product of the present invention contains a base, and a coating film formed by the coating material of the present invention applied over the base. Since the colored resin particles contain a polymer derived from pyrrole or a pyrrole derivative, the coated product of the present invention is conductive and antistatic.

Examples of the base include transparent base films, window glass, show windows, greenhouses, transparent food wrapping papers, transparent food wrapping films, showcases, illumination shades, posters, signboards, color photographs, medicine bottles, plastic bases such as polyethylene terephthalate (PET) bottles, glass, paper, metal films, plates made of various materials, yarns, and molded bodies made of various materials.

Examples of the method for applying the coating material include roll coating, bar coating, spray coating, air knife coating, flow coating, spin coating, dipping (dip coating), doctor blade method, electrostatic coating, letterpress printing, gravure printing, screen printing, pad printing, planographic printing, and flexographic printing.

The thickness of a coating film formed from these coating materials is preferably in a range of 1 to 100 µm.

### Optical Film and Anti-Glare Film

The coated product of the present invention can be used as a light-diffusing optical film, if a transparent base film is used as the base. The optical film of the present invention is obtained by applying the transparent coating material of the present invention over a transparent base film. That is to say, the optical film of the present invention contains a transparent base film, and a light-diffusing coating film (hereinafter, referred to as an "optical functional layer") formed over the transparent base film by applying the coating material of the present invention.

The coating material used in the optical film of the present invention preferably contains, in addition to the colored resin particles of the present invention, a resin binder that functions as a binder component of the colored resin particles, as the binder. The resin binder is a thermoplastic resin binder and/or a thermosetting resin binder.

The colored resin particles in the optical film of the present invention are mixed into a transparent coating material and are used to form an optical functional layer. There is no particular limitation on use of the optical functional layer, and it can be used as an optical functional layer for displays. For example, the optical functional layer can be used, more specifically, as a anti-glare layer, a hard coat layer, an antireflection layer, or the like in an optical film such as a surface film or a screen disposed on the surface of a display (in particular, high-definition image display). Since the colored resin particles contain a polymer derived from pyrrole or a pyrrole derivative, the optical functional layer is conductive, and thus, it can be used also as an antistatic layer in the above-mentioned various optical films.

The optical film of the present invention can be used as a anti-glare film, if the coating film has a rough face formed by the colored resin particles of the present invention. The anti-glare film of the present invention is obtained by applying the transparent coating material of the present invention over a transparent base film. That is to say, the anti-glare film of the present invention contains a transparent base film, and a anti-glare coating film (hereinafter, referred to as a "anti-glare layer") formed over the transparent base film by applying the coating material of the present invention.

Specifically, the mean particle diameter of the colored resin particles used in the optical film is preferably in a range of 0.5 to 10 µm. If the mean particle diameter of the colored resin particles is less than 0.5 µm, the particle diameter of the colored resin particles is smaller than the wavelength of the incident light, and light diffusion tends not to occur on the surfaces of the colored resin particles, so that the options to choose light that can be applied to an optical functional layer formed using the colored resin particles of the present invention are limited. Furthermore, if the mean particle diameter of the colored resin particles is less than 0.5 µm, an optical functional layer having sufficiently excellent optical function (anti-glare properties, black color reproduction, etc.) may not be obtained. If the mean particle diameter of the colored resin particles is more than 10 µm, the optical function (suppression of glare) of the optical functional layer becomes insufficient (e.g., glare tends to occur), and thus, the quality of a display or the like to which the optical film formed using the colored resin particles of the present invention is applied may deteriorate. When applying the optical film formed using the colored resin particles of the present invention to a display, the lower limit of the mean particle diameter of the colored resin particles is more preferably 0.8 µm in order to improve the display contrast, and the upper limit of the mean particle diameter of the colored resin particles is more preferably 8 µm in order to improve the contrast.

There is no particular limitation on the shape of the colored resin particles used in the optical film of the present invention, as long as it is any one of a recessed shape, a hemispherical shape, a double convex lens-like shape, and a mushroom-like shape, and it is preferably a double convex lens-like shape.

The amount of colored resin particles added to the optical film is preferably in a range of 0.5 to 20 parts by weight with respect to 100 parts by weight of resin binder. If the amount of colored resin particles added is less than 0.5 parts by weight with respect to 100 parts by weight of resin binder, the amount of colored resin particles added is small, and thus, the effects of the optical functional layer such as suppressing glare cannot be sufficiently obtained. If the amount of colored resin particles added is more than 20 parts by weight with respect to 100 parts by weight of resin binder, the amount of black portion in the optical film becomes too large, and thus, the transparency of the optical film becomes insufficient. The amount of colored resin particles added to the optical film is more preferably in a range of 1 to 10 parts by weight with respect to 100 parts by weight of resin binder.

There is no particular limitation on the transparent base film, and examples thereof include: plastic films such as polyester resin films, polycarbonate resin films, polyvinyl chloride resin films, polyethylene resin films, polypropylene resin films, and polystyrene resin films; and glass films. There is no particular limitation on the thickness of the transparent base film, and it is typically approximately 10 to 250 µm.

There is no particular limitation on the thickness of the optical functional layer in the optical film, and it is preferably in a range of 1 to 10 µm. There is no particular limitation on the thickness of the anti-glare layer in the anti-glare film, as long as a rough face resulting from the colored resin particles of the present invention is formed on the surface of the layer. The thickness is preferably in a range of 1 to 10 µm.

There is no particular limitation on the resin binder, as long as it is a transparent binder having a function of dispersing the colored resin particles, and examples thereof include ionizing radiation curable resins, which are resins that are cured with irradiation of ultraviolet rays or electron beams, solvent-drying resins, thermoplastic resins, and thermosetting resins.

Examples of the ionizing radiation curable resin include compounds having one or two or more unsaturated bonds, such as compounds having one or two or more (meth)acrylate-based functional groups ((meth)acryloyloxy groups).

Examples of the compound having one unsaturated bond include ethyl (meth)acrylate, 2-ethylhexyl (meth)acrylate, styrene, *α*-methylstyrene, and *N*-vinylpyrrolidone.

Examples of the compound having two or more unsaturated bonds include reaction products of a polyfunctional compound having a plurality of functional groups (hydroxyl groups, etc.) that can react with a carboxy group and a plurality of unsaturated carboxylic acids such as (meth)acrylic acids. Examples of the reaction product include poly(meth)acrylate of polyhydric alcohol. Examples of the poly(meth)acrylate of polyhydric alcohol include polymethylolpropane tri(meth)acrylate, hexanediol (meth)acrylate, tripropylene glycol di(meth)acrylate, diethyleneglycol di(meth)acrylate, pentaerythritol tri(meth)acrylate, dipentaerythritol hexa(meth)acrylate, 1,6-hexanediol di(meth)acrylate, and neopentylglycol di(meth)acrylate.

Examples of the ionizing radiation curable resin include not only the above-mentioned compounds but also polyester resin, polyether resin, acrylic resin, epoxy resin, urethane resin, alkyd resin, spiroacetal resin, polybutadiene resin, and polythiolpolyene resin, with an unsaturated double bond and having a relatively low molecular weight.

When using the colored resin particles of the present invention in a surface film disposed on the surface of a display, the resin binder is preferably made of an ultraviolet curable resin, which is a type of the ionizing radiation curable resin. When using the ultraviolet curable resin as the ionizing radiation curable resin, the coating material for forming the optical functional layer preferably contains a photopolymerization initiator.

Specific examples of the photopolymerization initiator include acetophenones, benzophenones, thioxanthones, Michler's benzoyl benzoate, *α*-amyloxim ester, thioxanthones, propiophenones, benzyls, benzoins (benzoin, benzoin methyl ether, etc.), acylphosphine oxides, aromatic diazonium salt, aromatic sulfonium salt, aromatic iodonium salt, metallocene compound, and benzoin sulfonate. Furthermore, the photopolymerization initiator is preferably used as a mixture with a photosensitizer. Specific examples of the photosensitizer include *n*-butylamine, triethylamine, and poly-*n*-butylphosphine. If the ionizing radiation curable resin is a resin having a radically polymerizable unsaturated group, acetophenones, benzophenones, thioxanthones, and benzoins are preferably used alone or in a combination as the photopolymerization initiator. Furthermore, if the ionizing radiation curable resin is a resin having a cationic polymerizable functional group, aromatic diazonium salt, aromatic sulfonium salt, aromatic iodonium salt, metallocene compound, and benzoin sulfonate are preferably used alone or in a combination as the photopolymerization initiator.

The amount of photopolymerization initiator added is preferably 0.1 to 10 parts by weight with respect to 100 parts by weight of ionizing radiation curable resin.

The ionizing radiation curable resin may be used together with a solvent-drying resin. Typical examples of the solvent-drying resin include a thermoplastic resin. Examples of the thermoplastic resin include commonly used various thermoplastic resins. If the solvent-drying resin is used together, coating film defectives on the coated face can be effectively prevented.

Specific examples of the thermoplastic resin used as a solvent-drying resin together with the ionizing radiation curable resin include styrene-based resins, (meth)acrylic-based resins, vinyl acetate-based resins, vinyl ether-based resins, halogen-containing resins, alicyclic olefin-based resins, polycarbonate-based resins, polyester-based resins, polyamide-based resins, cellulose derivatives, silicone-based resins, rubber, and elastomers. Typical examples of the thermoplastic resin preferably include resins that are non-crystalline and are soluble in organic solvent (in particular, common solvent in which a plurality of polymers and curable compounds can be dissolved). In particular, preferable examples thereof include resins having high moldability, film-forming performance, transparency, or weather resistance, such as styrene-based resins, (meth)acrylic-based resins, alicyclic olefin-based resins, polyester-based resins, cellulose derivatives (cellulose esters, etc.). Note that the solvent-drying resin may be used alone as the resin binder.

Examples of the thermosetting resin used as the resin binder include phenol resins, urea resins, diallylphthalate resins, melamine resins, guanamine resins, unsaturated polyester resins, polyurethane resins, epoxy resins, aminoalkyd resins, melamine-urea co-condensation resins, silicone resins, and polysiloxane resins. When using the thermosetting resin, as necessary, a cross-linking agent, a curing agent such as a polymerization initiator, a polymerization accelerator, solvent, a viscosity control agent, and the like may be used together.

Additives such as a leveling agent, an ultraviolet absorber, and an antioxidant may be added to the coating material for forming the optical functional layer. Furthermore, particles other than the colored resin particles may be added to the coating material for forming the optical functional layer.

The coating material for forming the optical functional layer can be prepared, for example, by dissolving or dispersing constituent components of the optical functional layer in appropriate solvent, thereby forming a solution or a dispersion (coating liquid).

Examples of the method for forming the optical functional layer include: a method in which a resin binder for forming the optical functional layer is dissolved or dispersed in appropriate solvent, and the colored resin particles are dispersed in the solvent, so that a liquid coating material (coating liquid) is prepared, after which the coating liquid is applied using a known method such as roll coating, bar coating, spray coating, or air knife coating, over a transparent base film and is then dried; and a method in which a resin binder for forming the optical functional layer is melted, and the colored resin particles and, as necessary, additives such as a pigment are added thereto, and the mixture is shaped into the form of sheets (molded into the form of sheets). Furthermore, the optical functional layer such as a anti-glare layer may be directly formed on the surface of a display apparatus or a screen using the above-described methods. Examples of the solvent used to form the optical functional layer include various types of solvent used in the coating material.

### External Preparation

The external preparation of the present invention contains the colored resin particles of the present invention. There is no particular limitation on the shape of the colored resin particles used in the external preparation of the present invention, as long as it is any one of a recessed shape, a hemispherical shape, a double convex lens-like shape, and a mushroom-like shape, preferably a double convex lens-like shape.

Examples of the external preparation into which the colored resin particles may be mixed include cosmetic materials and external medicines. Specific examples of the cosmetic materials into which the colored resin particles may be mixed include make-up cosmetic material compositions for the eyelashes, the eyebrows, or the skin, such as mascara, hair dyeing compositions, solid cosmetic materials such as blush and foundation, powder cosmetic materials such as body powder, and liquid cosmetic materials such as cream. If the colored resin particles according to the present invention are mixed thereinto, an external preparation such as a cosmetic material can be obtained that has a desired degree of coloring (e.g., black), has excellent adhesiveness, and does not easily come off.

Examples of the form of the external preparation such as a cosmetic material into which the colored resin particles may be mixed include forms such as lotion, gel, cream, powder, compressed powder, and stick, and forms used together with spray, foam, and aerosol.

Furthermore, in order to improve the optical functions or the touch feeling, an inorganic compound such as mica, talc, or organic hectorite, iron oxide, titanium oxide, and the like may be added to the external preparation such as a cosmetic material. In the case of a liquid external preparation such as a liquid cosmetic material, there is no particular limitation on the liquid medium, and examples thereof include water, alcohol, hydrocarbon, silicone oil, vegetable fat and oils, and animal fat and oils. The external preparation such as a cosmetic material may be further provided with various functions by adding not only the above-described other components but also other components commonly used in an external preparation such as cosmetic, such as wax (paraffin wax, carnauba wax, beeswax, etc.), coating film-forming polymer (polyvinyl laurate, vinyl acetate / allyl stearate copolymer, etc.), starch (e.g., rice starch), ester-based solvent (e.g., propylene carbonate), preservative, liquid paraffin (e.g., isododecane), moisturizing agent, anti-inflammatory agent, whitening agents, UV care agent, germicide, antiperspirant, algefacient, fragrance, soap (e.g., sodium oleate).

### Examples

Hereinafter, the present invention will be more specifically described by way of examples, but the present invention is not limited to these examples.

First, the measuring methods and the calculation methods in the examples will be described.

### Method for Measuring the Weight Average Molecular Weight

The weight average molecular weight (Mw) was measured using gel permeation chromatography (GPC). Note that the weight average molecular weight refers to the polystyrene(PS)-equivalent weight average molecular weight. Specifically, the weight average molecular weight was measured as follows.

First, 50 mg of sample was dissolved in 10 ml of tetrahydrofuran (THF), and filtered with a 0.45-µm non-aqueous chromatodisc, and the obtained material was measured using gel permeation chromatography. The conditions of the chromatography were as below.
Gel permeation chromatograph: brand name "gel permeation chromatograph HLC-8020" manufactured by Tosoh Corporation
Column: brand name "TSKgel GMH-XL-L" manufactured by Tosoh Corporation, φ 7.8 mm x 30 cm x 2 columns
Column temperature: 40°C
Carrier gas: tetrahydrofuran (THF)
Carrier gas flow rate: 1 ml /min
Injection and pump temperatures: 35°C
Detection: RI
Injection amount: 100 microliter
Standard polystyrene for calibration curve: brand name "Shodex (registered trademark)" manufactured by Showa Denko K.K., with a weight average molecular weight of 1030000, and products manufactured by Tosoh Corporation with weight average molecular weights of 5480000, 3840000, 355000, 102000, 37900, 9100, 2630, and 870

### Method for Measuring the Mean Particle diameter of the Seed Particles

The mean particle diameter of the seed particles was measured using an LS230 manufactured by Beckman Coulter. Specifically, 0.1 g of particles and 10 ml of 0.1% nonionic surfactant solution were charged, and mixed for two seconds using a touch mixer "TOUCHMIXER MT-31" manufactured by Yamato Scientific Co., Ltd. Subsequently, the test tube was placed in a commercially available ultrasonic cleaner "ULTRASONIC CLEANER VS-150" manufactured by Velvo-Clear, and its content was dispersed for 10 minutes. The dispersed material was measured under the irradiation of ultrasonic waves, using an LS230 manufactured by Beckman Coulter. The optical model at that time was adjusted according to the refractive index of the produced particles.

### Method for Measuring Lengths A to I of Various Portions of Irregular Particles

The lengths A to I of various portions of an irregular particles were measured as follows.

With observation of 30 freely picked up irregular particles using a scanning electron microscope JSM-6360LV (manufactured by JEOL Ltd.) at 5000 to 10000 magnification, various portions of each irregular particle were measured, and their average values were taken as lengths A to I.

### Method for Measuring the Sphere-Equivalent Volume Mean Particle diameter of Irregular Particles and Colored Resin Particles and the Mean Particle diameter of Spherical Particles

A pore having a pore size of 50 to 280 µm was filled with an electrolyte solution, and the volume of particles was obtained based on a change in the electrical conductivity of the electrolyte solution at the time when the particles passed through the electrolyte solution, and the mean particle diameter (the sphere-equivalent volume mean particle diameter of the irregular particles and the colored resin particles and the mean particle diameter of the spherical particles) was calculated. Specifically, the measured mean particle diameter is a volume mean particle diameter (arithmetic mean value) measured using a COULTER MULTISIZER II manufactured by Beckman Coulter. Note that at the time of measurement, the measurement was performed after calibration using an aperture suited for the particle diameter of particles that were to be measured, according to REFERENCE MANUAL FOR THE COULTER MULTISIZER (1987) issued by Coulter Electronics Limited.

Specifically, 0.1 g of particles and 10 ml of 0.1% nonionic surfactant solution were charged into a commercially available glass test tube. The charged material was mixed for two seconds using a touch mixer "TOUCHMIXER MT-31" manufactured by Yamato Scientific Co., Ltd., after which the content of the test tube was subjected to preliminary dispersion for 10 seconds using a commercially available ultrasonic cleaner "ULTRASONIC CLEANER VS-150" manufactured by Velvo-Clear. The dispersion was added dropwise using a dropper with gentle stirring to a beaker filled with ISOTON II (measurement electrolyte manufactured by Beckman Coulter), attached to the main body, and a value indicated by a concentration meter on the screen of the main body was adjusted to approximately 10%. Next, the aperture size, the current, the gain, and the polarity were input to the MULTISIZER II main body according to REFERENCE MANUAL FOR THE COULTER MULTISIZER (1987) issued by Coulter Electronics Limited, and the measurement was performed in a manual mode. During the measurement, the content of the beaker was gently stirred such that air bubbles were not formed therein, and the measurement was ended when 100000 particles were measured.

### Method for Measuring the Coefficient of Variation (CV) of the Colored Resin Particles

The coefficient of variation (CV) in the particle diameter of the colored resin particles was calculated using the following equation.

Coefficient of variation of particle diameter of colored resin particles = (Standard deviation of volume-based particle diameter distribution of colored resin particles / sphere-equivalent volume mean particle diameter of colored resin particles) x 100

### Seed Particle-Forming Emulsion Synthesis Example 1

After 600 g of water, 100 g of methyl methacrylate, and 0.5 g of *n*-dodecyl mercaptan were charged into a separable flask equipped with a stirrer, a thermometer, and a reflux condenser, nitrogen purge was performed and the temperature was increased to 70°C with stirring. The temperature inside the flask was kept at 70°C, and 0.5 g of potassium persulfate was added as the polymerization initiator to the stirred content, after which polymerization reaction was allowed to occur for 8 hours to form an emulsion. The obtained emulsion had a solid content of 14%. The solid content was made of spherical particles having a mean particle diameter of 0.4 µm and a weight average molecular weight of 600000.

### Seed Particle-Forming Emulsion Synthesis Example 2

After 600 g of water and 100 g of methyl methacrylate were charged into a separable flask equipped with a stirrer, a thermometer, and a reflux condenser, nitrogen purge was performed and the temperature was increased to 70°C with stirring. The temperature inside the flask was kept at 70°C, and 0.5 g of potassium persulfate was added as the polymerization initiator to the stirred content, after which polymerization reaction was allowed to occur for 12 hours to form an emulsion. The obtained emulsion had a solid content of 14%. The solid content was made of spherical particles having a mean particle diameter of 0.43 µm and a weight average molecular weight of 820000.

### Seed Particle-Forming Emulsion Synthesis Example 3

After 600 g of water, 100 g of methyl methacrylate, and 1.1 g of *n*-octyl mercaptan were charged into a separable flask equipped with a stirrer, a thermometer, and a reflux condenser, nitrogen purge was performed and the temperature was increased to 70°C with stirring. The temperature inside the flask was kept at 70°C, and 0.5 g of potassium persulfate was added as the polymerization initiator to the stirred content, after which polymerization reaction was allowed to occur for 12 hours to form an emulsion. The obtained emulsion had a solid content of 14%. The solid content was made of spherical particles having a mean particle diameter of 0.43 µm and a weight average molecular weight of 30000.

### Seed Particle-Production Example 1

After 550 g of water, 70 g of emulsion obtained in Synthesis Example 1, 100 g of isobutyl methacrylate, and 0.3 g of *n*-dodecyl mercaptan were charged into a separable flask equipped with a stirrer, a thermometer, and a reflux condenser, nitrogen purge was performed and the temperature was increased to 70°C with stirring. The temperature inside the flask was kept at 70°C, and 0.5 g of potassium persulfate was added as the polymerization initiator, after which polymerization reaction was allowed to occur for 8 hours. The obtained emulsion had a solid content of 14%, and the solid content was made of spherical particles (seed particles) having a mean particle diameter of 1.1 µm and a weight average molecular weight of 610000.

### Seed Particle-Production Example 2

After 550 g of water, 70 g of emulsion obtained in Synthesis Example 2, and 100 g of isobutyl methacrylate were charged into a separable flask equipped with a stirrer, a thermometer, and a reflux condenser, nitrogen purge was performed and the temperature was increased to 70°C with stirring. The temperature inside the flask was kept at 70°C, and 0.5 g of potassium persulfate was added as the polymerization initiator, after which polymerization reaction was allowed to occur for 8 hours. The obtained emulsion had a solid content of 14%, and the solid content was made of spherical particles (seed particles) having a mean particle diameter of 1.0 µm and a weight average molecular weight of 830000.

### Resin Particle-Production Example 1

In a 5-L reactor equipped with a stirrer and a thermometer, 600 g of methyl methacrylate, 300 g of ethylene glycol dimethacrylate, and 100 g of poly(ethyleneglycol-propyleneglycol) monomethacrylate (brand name: Blemmer (registered trademark) 50PEP-300/ manufactured by NOF Corporation, in General Formula above, R₁ = CH₃, R₂ = C₂H₄, R₃ = C₃H₆, R₄ = H, and this product is a mixture with an average m of 3.5 and an average n of 2.5) as the polymerizable vinyl-based monomer, and 6 g of 2,2'-azobisisobutyronitrile as the polymerization initiator were charged and mixed. The obtained mixture was mixed with 1 L of ion-exchanged water containing 10 g of sodium sulfosuccinate as the surfactant, and treated at 8000 rpm for 10 minutes using a TK homo mixer (manufactured by Primix Corporation) to form an aqueous emulsion. Then, 360 g of seed particle-containing emulsion having a mean particle diameter of 1.1 µm obtained in Seed Particle-Production Example 1 was added to this aqueous emulsion with stirring.

After the stirring was continued for 3 hours, the aqueous emulsion was observed using an optical microscope. The observation showed that the polymerizable vinyl-based monomer in the aqueous emulsion was absorbed by the seed particles (swelling factor: about 20 times). Subsequently, 2000 g of aqueous solution containing 40 g of polyvinyl alcohol (PVA-224E manufactured by Kuraray Co., Ltd.) dissolved therein was charged as the dispersion stabilizer into the reactor, and polymerization was allowed to occur with stirring at 60°C for 6 hours. Resin particles were isolated from the aqueous emulsion after the polymerization. The obtained resin particles (core particles) were observed using a scanning electron microscope. The observation showed that the particles were irregular particles having a hemispherical shape (A = 2.92 µm, F = 1.71 µm). Furthermore, the irregular particles had a sphere-equivalent volume mean particle diameter of 2.60 µm.

### Resin Particle-Production Example 2

Resin particles were obtained as in Resin Particle-Production Example 1, except that 700 g of methyl methacrylate and 300 g of ethylene glycol dimethacrylate were used as the polymerizable vinyl-based monomer, and that poly(ethyleneglycol-propyleneglycol) monomethacrylate was not used. The obtained resin particles were observed using a scanning electron microscope. The observation showed that the particles were irregular particles having a double convex lens-like shape (A = 2.88 µm, H = 1.27 µm, I = 0.64 µm). Furthermore, the irregular particles had a sphere-equivalent volume mean particle diameter of 2.61 µm.

### Resin Particle-Production Example 3

Resin particles were obtained as in Resin Particle-Production Example 1, except that the swelling factor was set to about 40 times. The obtained resin particles were observed using a scanning electron microscope. The observation showed that the particles were irregular particles with a recessed cross-section, having one cut-out portion radially extending therethrough (A = 3.45 µm, B = 1.15 µm, C = 1.74 µm). Furthermore, the irregular particles had a sphere-equivalent volume mean particle diameter of 3.10 µm.

### Resin Particle-Production Example 4

Resin particles were obtained as in Resin Particle-Production Example 1, except that the seed particle-containing emulsion produced in Seed Particle-Production Example 2 was-used. The obtained resin particles were observed using a scanning electron microscope. The observation showed that the particles were irregular particles having a mushroom-like shape (A = 3.26 µm, D1 = 1.43 µm, D2 = 1.87 µm, E = 1.80 µm). Furthermore, the irregular particles had a sphere-equivalent volume mean particle diameter of 2.71 µm.

### Resin Particle-Production Example 5

Resin particles were obtained as in Resin Particle-Production Example 1, except that 700 g of methyl methacrylate and 300 g of ethylene glycol dimethacrylate were used as the polymerizable vinyl-based monomer, that 41.6 g of emulsion obtained in Seed Particle-Forming Emulsion Synthesis Example 3 was used instead of the seed particle-containing emulsion obtained in Seed Particle-Production Example 1, and that poly(ethyleneglycol-propyleneglycol) monomethacrylate was not used. The obtained resin particles were observed using a scanning electron microscope. The observation showed that the particles were spherical particles. The resin particles had a mean particle diameter of 2.51 µm.

### Example 1

A suspension obtained by dispersing 50 g of resin particles (hemispherical irregular particles) obtained in Resin Particle-Production Example 1 in 50 g of isopropanol was added to a solution obtained by dissolving 20 g of potassium persulfate (1.0 molar equivalents with respect to pyrrole) in 300 g of water, and the mixture was stirred. This suspension was cooled down to 5°C, and a solution made of 5 g of pyrrole and 50 g of isopropanol was further added thereto, after which polymerization was allowed to occur with stirring for 3 hours. Subsequently, filtering was performed to obtain a solid content. The solid content was collected, washed with water and isopropanol, and dried using a vacuum dryer at 60°C for 12 hours, to form black colored resin particles surface-coated with a pyrrole polymer.

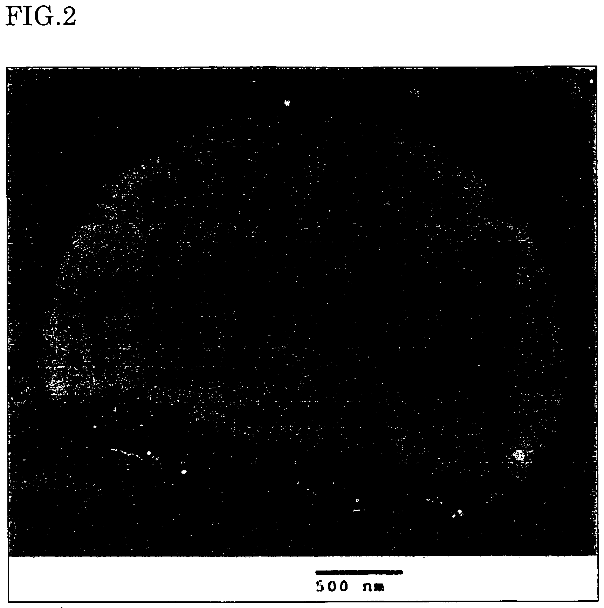

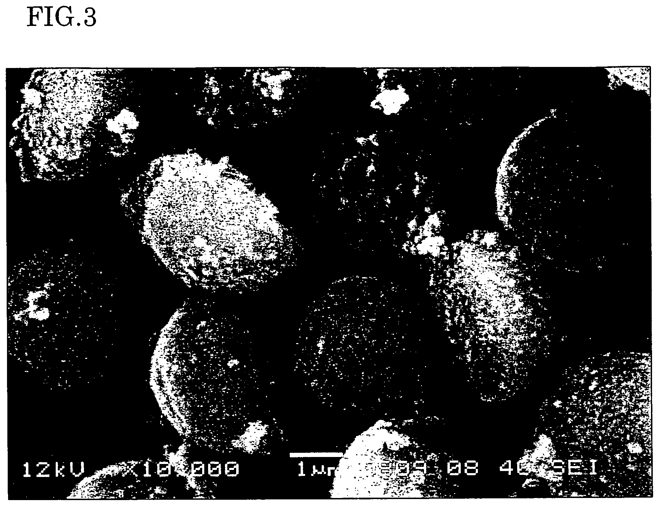

The obtained colored resin particles were observed using a transmission electron microscope. The observation showed that the entire surfaces of the resin particles were uniformly coated with a polymer derived from pyrrole (nitrogen-containing aromatic compound). Furthermore, the obtained colored resin particles were observed using a scanning electron microscope. The observation showed that the particles were hemispherical irregular particles. FIG. 2 shows a cross-sectional photograph of a colored resin particle according to Example 1 taken by the transmission electron microscope. FIG. 3 shows a photograph of the colored resin particles according to Example 1 taken by the scanning electron microscope.

The obtained colored resin particles had a coefficient of variation (CV) in the particle diameter of 11.3%.

### Example 2

Black colored resin particles surface-coated with a pyrrole polymer were obtained as in Example 1, except that 50 g of resin particles (double convex lens-like irregular particles) of Resin Particle-Production Example 2 was used instead of the resin particles of Resin Particle-Production Example 1.

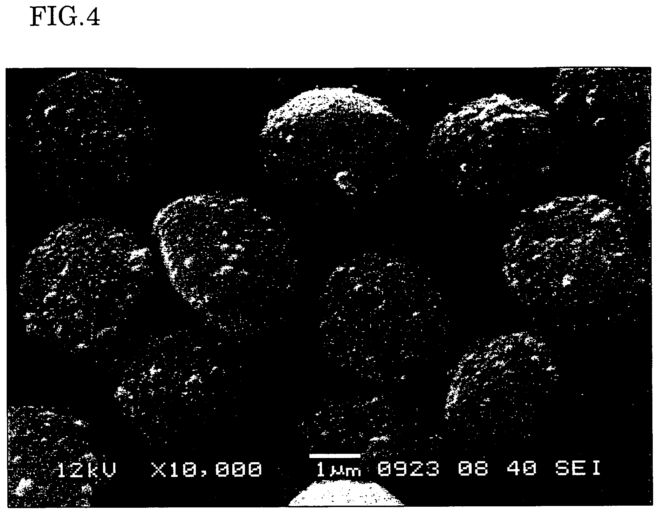

The obtained colored resin particles were observed using a scanning electron microscope. The observation showed that the particles were double convex lens-like irregular particles. FIG. 4 shows a photograph of the colored resin particles according to Example 2 taken by the scanning electron microscope.

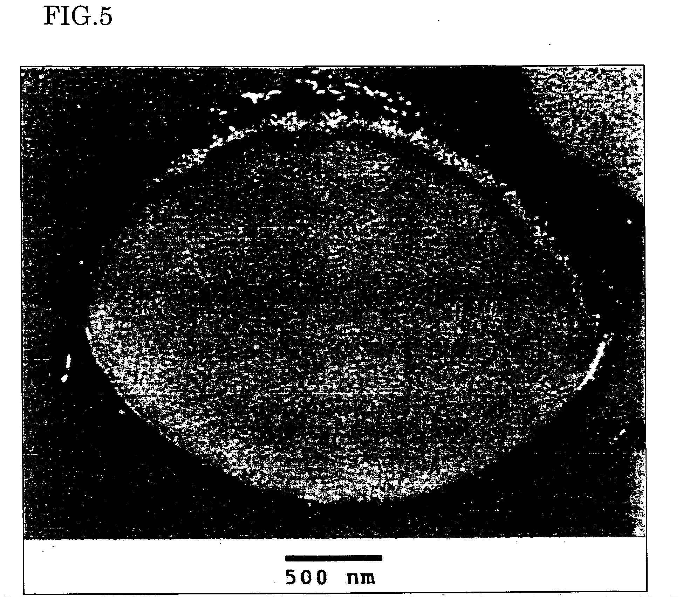

Furthermore, the obtained colored resin particles were observed using a transmission electron microscope. The observation showed that the entire surfaces of the resin particles were uniformly coated with a polymer derived from pyrrole (nitrogen-containing aromatic compound). FIG. 5 shows a cross-sectional photograph of a colored resin particle according to Example 2 taken by the transmission electron microscope.

The obtained colored resin particles had a coefficient of variation (CV) in the particle diameter of 10.8%.

### Example 3

Black colored resin particles surface-coated with a pyrrole polymer were obtained as in Example 1, except that 50 g of resin particles (irregular particles with a recessed cross-section, having one cut-out portion radially extending therethrough) of Resin Particle-Production Example 3 was used instead of the resin particles of Resin Particle-Production Example 1.

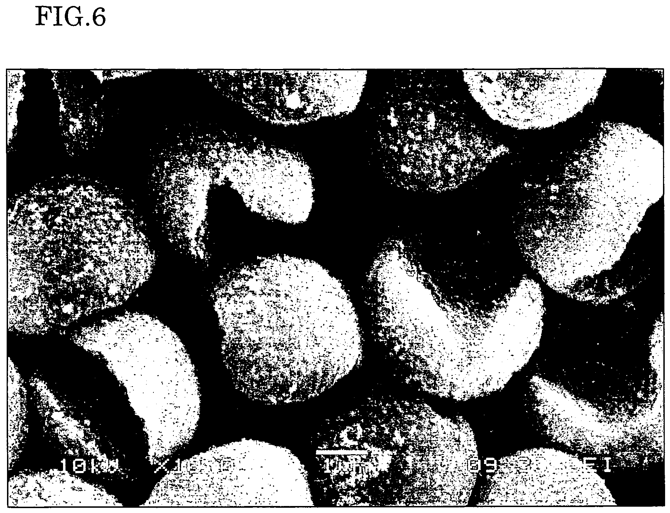

The obtained colored resin particles were observed using a scanning electron microscope. The observation showed that the particles were irregular particles with a recessed cross-section, having one cut-out portion radially extending therethrough. FIG. 6 shows a photograph of the colored resin particles according to Example 3 taken by the scanning electron microscope.

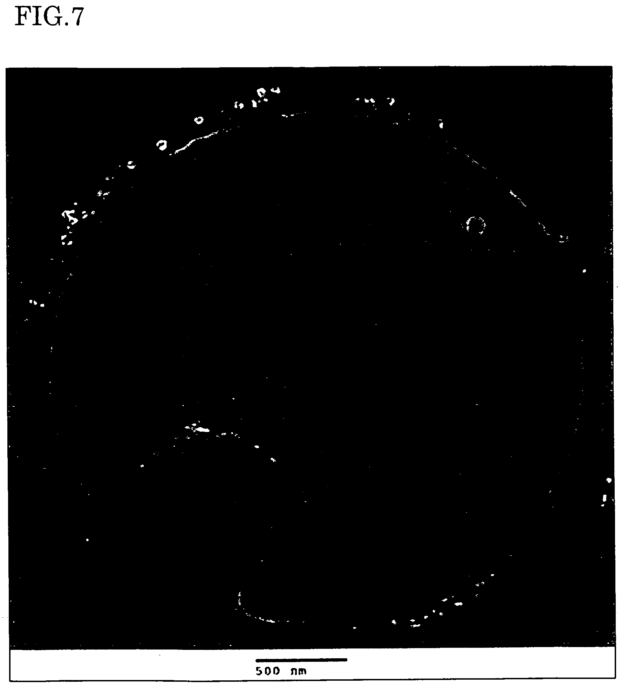

Furthermore, the obtained colored resin particles were observed using a transmission electron microscope. The observation showed that the entire surfaces of the resin particles were uniformly coated with a polymer derived from pyrrole (nitrogen-containing aromatic compound). FIG. 7 shows a cross-sectional photograph of a colored resin particle according to Example 3 taken by the transmission electron microscope.

The obtained colored resin particles had a coefficient of variation (CV) in the particle diameter of 11.6%.

### Example 4

Black colored resin particles surface-coated with a pyrrole polymer were obtained as in Example 1, except that 50 g of resin particles (mushroom-like irregular particles) of Resin Particle-Production Example 4 was used instead of the resin particles of Resin Particle-Production Example 1.

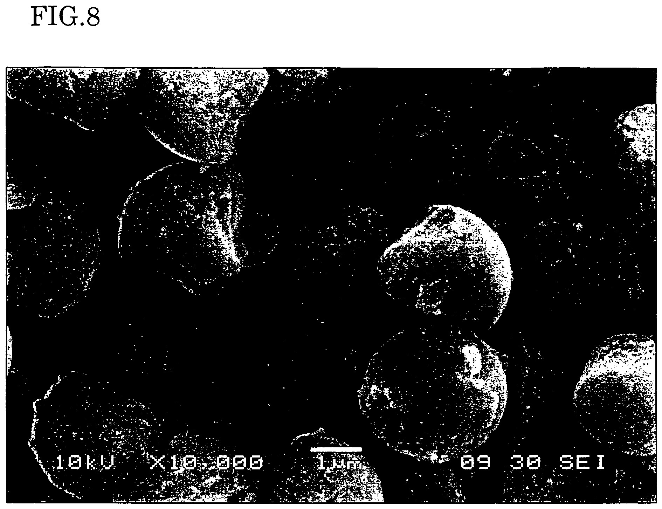

The obtained colored resin particles were observed using a scanning electron microscope. The observation showed that the particles were mushroom-like irregular particles. FIG. 8 shows a photograph of the colored resin particles according to Example 4 taken by the scanning electron microscope.

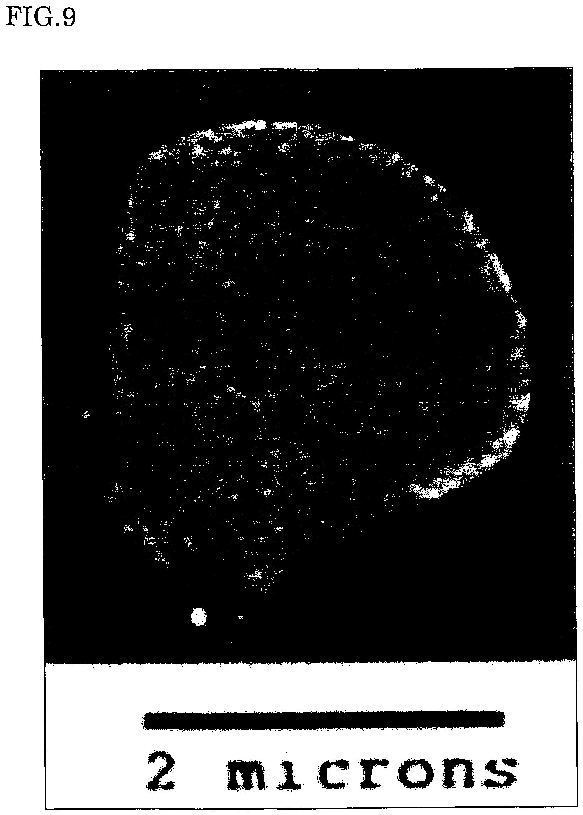

Furthermore, the obtained colored resin particles were observed using a transmission electron microscope. The observation showed that the entire surfaces of the resin particles were uniformly coated with a polymer derived from pyrrole (nitrogen-containing aromatic compound). FIG. 9 shows a cross-sectional photograph of a colored resin particle according to Example 4 taken by the transmission electron microscope.

The obtained colored resin particles had a coefficient of variation (CV) in the particle diameter of 10.9%.

Comparative Example 1: Method for Producing Spherical Colored Resin Particles

Black spherical colored resin particles surface-coated with a pyrrole polymer were obtained as in Example 1, except that 50 g of resin particles (spherical particles) of Resin Particle-Production Example 5 was used instead of the resin particles of Resin Particle-Production Example 1.

The obtained colored resin particles had a coefficient of variation (CV) in the particle diameter of 10.8%.

Table 1 shows a summary of Examples 1 to 4 and Comparative Example 1.

**Table 1**

| | | Ex. 1 | Ex. 2 | Ex. 3 | Ex. 4 | Com. Ex. 1 |
|---|---|---|---|---|---|---|
| | Resin Particle-Production Example No. | 1 | 2 | 3 | 4 | 5 |
| | Methyl methacrylate (g) | 600 | 700 | 600 | 600 | 700 |
| | Ethylene glycol dimethacrylate (g) | 300 | 300 | 300 | 300 | 300 |
| Resin particles | Poly(ethyleneglycol-propyleneglycol) monomethacrylate (g) | 100 | - | 100 | 100 | - |
| | 2,2'-Azobisisobutyronitrile (g) | 6 | 6 | 6 | 6 | 6 |
| | Swelling factor | 20 | 20 | 40 | 20 | 171 |
| | Production Example No. of seed particles charged | 1 | 1 | 1 | 2 | Seed Particle -Forming Emulsion Synthesis Example 3 |
| | Amount of seed particle-containing emulsion added (g) | 360 | 360 | 360 | 360 | 41.6 |
| | Shape of obtained particles | Hemisphere | Double convex lens | Recessed cross-section | Mushroom | Sphere |
| Colored resin particles | Potassium persulfate (g) | 20 | 20 | 20 | 20 | 20 |
| | Isopropanol (g) | 50 | 50 | 50 | 50 | 50 |
| | Pyrrole (g) | 5 | 5 | 5 | 5 | 5 |
| | Amount of persulfate added with respect to pyrrole (molar equivalent) | 1.0 | 1.0 | 1.0 | 1.0 | 1.0 |
| | Resin particles charged (g) | 50 | 50 | 50 | 50 | 50 |
| | CV (%) | 11.3 | 10.8 | 11.6 | 10.9 | 10.8 |
| | Note (photograph) | FIGS. 2 and 3 | FIGS. 4 and 5 | FIGS. 6 and 7 | FIGS. 8 and 9 | - |

### Example 5

### Production of Hard Coat Layer Coating Liquid

First, 200 parts by weight of acrylic monomer (brand name "KAYARAD (registered trademark) DPHA", dipentaerythritol hexaacrylate, manufactured by Nippon Kayaku Co., Ltd.) as the resin binder, 25 parts by weight of photopolymerization initiator (Irgacure (registered trademark) 184 (manufactured by BASF Japan Ltd.), 1-hydroxy-cyclohexyl-phenyl-ketone), 110 parts by weight of propylene glycol monomethyl ether as the solvent, 110 parts by weight of ethyl acetate as the solvent, and 20 parts by weight of double convex lens-like colored resin particles (sphere-equivalent volume mean particle diameter: 2.61 µm) obtained in Example 2 were mixed with stirring to form a hard coat layer coating liquid as a coating material.

### Production of Anti-Glare Hard Coat Film

The hard coat layer coating liquid was applied over a PET film having a thickness of 188 µm as the transparent base film using a micro gravure coater, to form a coating layer (layer of the hard coat layer coating liquid). Then, the coating layer was dried at 90°C. Subsequently, the coating layer was cured by irradiating the coating layer with ultraviolet rays using an ultraviolet lamp at an illuminance of the irradiating portion of 0.1 W/cm² and an irradiation dose of 0.1 J/cm². Accordingly, a anti-glare hard coat layer having a thickness of 5 µm was formed on the PET film, and a anti-glare hard coat film including the PET film and the anti-glare hard coat layer was produced as a anti-glare film, which was a type of optical film (a type of coated product).

### Example 6

A hard coat film was produced as in Example 5, except that the use amount of double convex lens-like colored resin particles obtained in Example 2 was changed from 20 parts by weight to 16 parts by weight.

### Example 7

A hard coat film was produced as in Example 5, except that the use amount of double convex lens-like colored resin particles obtained in Example 2 was changed from 20 parts by weight to 12 parts by weight.

### Comparative Example 2

A hard coat film was produced as in Example 6, except that the double convex lens-like resin particles obtained in Resin Particle-Production Example 2 were used instead of the double convex lens-like colored resin particles obtained in Example 2.

### Comparative Example 3

A hard coat film was produced as in Example 7, except that the spherical colored resin particles obtained in Comparative Example 1 were used instead of the double convex lens-like colored resin particles obtained in Example 2.

### Measurement of the Haze and the Total Light Transmittance

The haze and the total light transmittance of each of the hard coat films obtained in Examples 5 to 7 and Comparative Examples 2 and 3 were measured using a haze meter NDH-2000. The haze was measured according to JIS K 7136, and the total light transmittance was measured according to JIS K 7361-1. Table 2 shows the measurement results.

### Evaluation regarding Glare

Each of the hard coat films obtained in Examples 5 to 7 and Comparative Examples 2 and 3 was placed on the surface of a tablet terminal displaying images such that the coated face (face on the side having the anti-glare hard coat layer) was in contact with the surface of the tablet terminal. Visual evaluation was performed regarding glare of the images at that time. A rating of "Good" was given if there was no glare, "Fair" was given if there was slight glare, and "Poor" was given if there was a large amount of glare. Table 2 shows the evaluation results.

**Table 2**

| Evaluation | Ex. 5 | Ex. 6 | Ex. 7 | Com. Ex. 2 | Com. Ex. 3 |
|---|---|---|---|---|---|
| Haze (%) | 51.1 | 40.8 | 25.3 | 44 | 28.4 |
| Total light transmittance (%) | 83.9 | 84 | 84.8 | 87.7 | 84.9 |
| Glare | Good | Good | Good | Fair | Fair |

Comparison between Examples 5 to 7 and Comparative Examples 2 and 3 shows that the anti-glare films obtained using the irregular colored resin particles of the present invention can suppress glare of display, without significantly decreasing the brightness of display by a display apparatus, and without significantly reducing the effect of preventing specular reflection of external light on a display face, compared with the anti-glare films obtained using spherical transparent resin particles and spherical colored resin particles. Furthermore, it seems that, since the anti-glare films of the present invention can suppress glare of display, they can suppress a reduction in the display contrast due to the anti-glare films.

### Example 8

Mascara was produced following a preparation process commonly used for preparing cosmetics, with the formulation below.

| | |
|---|---|
| Paraffin wax | 2 g |
| Carnauba wax | 4 g |
| Beeswax | 8 g |
| Polyvinyl laurate | 0.8 g |
| ("Mexomer PP" manufactured by Chimex S.A.) | |
| Vinyl acetate / allyl stearate copolymer (65/35) | 2 g |
| Rice starch | 1 g |
| Organic hectorite (manufactured by Elementis Specialties, Inc.) | 4 g |
| (brand name "BENTONE (registered trademark)") | |
| Propylene carbonate | 2 g |
| Double convex lens-like colored resin particles obtained in Example 2 | 4 g |
| Preservative | appropriate amount |
| Isododecane | 100 g in appropriate amount |

As an application tool for the mascara, a brush formed by twisting fiber with a wire, commonly used for cosmetic materials for the eyelashes, having a total length of the application portion of 25 mm, a maximum diameter of 5.0 mm, a tip diameter of 3.5 mm, a base diameter of 4.0 mm, and a shape of an R55 (radius of curvature: 55 mm) bow was used.

### Comparative Example 4

Mascara was produced as in Example 8, except that the spherical colored resin particles obtained in Comparative Example 1 were used instead of the double convex lens-like colored resin particles obtained in Example 2.

### (1) Curl Up Effect

Next, 10 testers (selected panelists) applied the mascaras of Example 8 and Comparative Example 4 to their eyelashes using the above-described application tool, and visually evaluated the state of the eyelashes immediately after the application. The curl up effect of each mascara was evaluated through the number of testers who rated the curl up effect excellent. A rating of "Good" was given if the number of testers who rated the curl up effect excellent was 8 to 10, "Fair" was given if the number of testers who rated the curl up effect excellent was 5 to 7, and "Poor" was given if the number of testers who rated the curl up effect excellent was 0 to 4. Table 3 shows the obtained results.

### (2) Makeup Lasting Power

Furthermore, 10 testers applied the mascaras of Example 8 and Comparative Example 4 to their eyelashes using the above-described application tool, and visually evaluated the state of the eyelashes after 8 hours. The makeup lasting power of each mascara was evaluated through the number of testers who rated the smudge-proof properties excellent. A rating of "Good" was given if the number of testers who rated the smudge-proof properties excellent was 8 to 10, "Fair" was given if the number of testers who rated the smudge-proof properties excellent was 5 to 7, and "Poor" was given if the number of testers who rated the smudge-proof properties excellent was 0 to 4. Table 3 shows the obtained results.

**Table 3**

| | Shape of colored resin particles | Curl up effect | Makeup lasting power |
|---|---|---|---|
| Ex. 8 | Double convex lens | Good | Good |
| Com. Ex. 4 | Sphere | Fair | Fair |

The results of Example 8 and Comparative Example 4 show that the external preparations using the colored resin particles of the present invention have excellent adhesiveness to the eyelashes, and do not easily come off, compared with the external preparations using spherical colored resin particles.

### Example 9

### Production of Compound

First, 100 parts by weight of aromatic polycarbonate resin (brand name "TARFLON (registered trademark) A2500" manufactured by Idemitsu Kosan Co., Ltd.) obtained by reacting 2,2-bis(4-hydroxyphenyl)propane with carbonate precursor, as the base resin, and 5 parts by weight of double convex lens-like colored resin particles obtained in Example 2 as the polymer particles (light diffusing agent) were mixed using a Henschel mixer for 15 minutes. The obtained mixture was extruded using a single screw extruder (brand name "R50" manufactured by Hoshi Plastics) at a temperature of 250 to 280°C and an extrusion rate of 10 to 25 kg/hr, and the extruded mixture was cooled down with water, to form a compound as a resin composition. The compound was cut using a pelletizer, to form a light-diffusing resin composition in the form of pellets.

### Production of Molded Body

The obtained light-diffusing resin composition in the form of pellets was pre-dried at 120°C for 5 hours, so that water was sufficiently removed. Subsequently, the light-diffusing resin composition was subjected to injection molding using an injection molding machine (brand name "K-80" manufactured by Kawaguchi, Ltd.) at a cylinder temperature of 255 to 280°C, to form a light-diffusing plate (a type of molded body) having a thickness of 2 mm, a width of 50 mm, and a length of 100 mm, as a light-diffusing member.

### Comparative Example 5

### Production of Compound

First, 100 parts by weight of aromatic polycarbonate resin (brand name "TARFLON (registered trademark) A2500" manufactured by Idemitsu Kosan Co., Ltd.) obtained by reacting 2,2-bis(4-hydroxyphenyl)propane with carbonate precursor, as the base resin, and 5 parts by weight of spherical colored resin particles obtained in Comparative Example 1 as the polymer particles (light diffusing agent) were mixed using a Henschel mixer for 15 minutes. The obtained mixture was extruded using a single screw extruder (brand name "R50" manufactured by Hoshi Plastics) at a temperature of 250 to 280°C and an extrusion rate of 10 to 25 kg/hr, and the extruded mixture was cooled down with water, to form a compound as a resin composition. The compound was cut using a pelletizer, to form a light-diffusing resin composition in the form of pellets.

### Production of Molded Body

The obtained light-diffusing resin composition in the form of pellets was pre-dried at 120°C for 5 hours, so that water was sufficiently removed. Subsequently, the light-diffusing resin composition was subjected to injection molding using an injection molding machine (brand name "K-80" manufactured by Kawaguchi, Ltd.) at a cylinder temperature of 255 to 280°C, to form a light-diffusing plate (a type of molded body) having a thickness of 2 mm, a width of 50 mm, and a length of 100 mm, as a light-diffusing member.

In order to evaluate the conductivity, the surface specific resistance (unit: Ω) of the light-diffusing plates obtained in Example 9 and Comparative Example 5 was measured at an applied voltage of 500 V for 1 minute using an high resistivity meter (brand name "Hiresta UP" manufactured by Mitsubishi Chemical Analytech Co., Ltd.) according to JIS K6911. The total light transmission of the light-diffusing plates obtained in Example 9 and Comparative Example 5 was measured according to JIS K7136. Table 4 shows the measurement results of the surface specific resistance and the total light transmittance.

**Table 4**

| | Ex. 9 | Com. Ex. 5 |
|---|---|---|
| Surface specific resistance | 2.0 × 10¹⁰ (Ω) | 3.0 × 10¹⁰ (Ω) |
| Total light transmittance | 3.0% | 2.1% |

Comparison between the results of Example 9 and Comparative Example 5 shows that the molded body using the irregular colored resin particles of the present invention has a higher total light transmittance and a lower surface specific resistance than those of the molded body using spherical colored resin particles.

## Claims

1. Colored resin particles surface-coated with a polymer derived from a nitrogen-containing aromatic compound, wherein each of the particles has an irregular shape with a circular appearance when viewed from a direction in which a projected area thereof becomes maximum, and with a non-circular appearance when viewed from a direction in which the projected area thereof becomes minimum, wherein:
the nitrogen-containing aromatic compound is pyrrole or a pyrrole derivative;
the resin particles have any one of a recessed shape, a hemispherical shape, a mushroom-like shape, or a double convex lens-like shape; and
the resin particles having the double convex lens-like shape are obtainable by allowing seed particles to absorb a polymerizable vinyl-based monomer in an aqueous emulsion, the polymerizable vinyl-based monomer containing 5 to 50% by weight of a polyfunctional monomer in which one molecule has two or more polymerizable vinyl groups, and by polymerizing the absorbed polymerizable vinyl-based monomer.

2. The colored resin particles according to claim 1, wherein the resin particles are obtained by polymerizing a polymerizable vinyl-based monomer containing (meth)acrylic acid ester having an alkylene oxide group.

3. A method for producing colored resin particles, comprising:
a step of obtaining resin particles each of which has an irregular shape with a circular appearance when viewed from a direction in which a projected area thereof becomes maximum, and with a non-circular appearance when viewed from a direction in which the projected area thereof becomes minimum, by polymerizing a polymerizable vinyl-based monomer; and
a step of coating the surfaces of the obtained resin particles with a polymer derived from a nitrogen-containing aromatic compound, wherein:
the nitrogen-containing aromatic compound is pyrrole or a pyrrole derivative;
the resin particles have any one of a recessed shape, a hemispherical shape, a mushroom-like shape, or a double convex lens-like shape; and
the step of obtaining resin particles having the double convex lens-like shape comprises allowing seed particles to absorb a polymerizable vinyl-based monomer in an aqueous emulsion, the polymerizable vinyl-based monomer containing 5 to 50% by weight of a polyfunctional monomer in which one molecule has two or more polymerizable vinyl groups, and polymerizing the absorbed polymerizable vinyl-based monomer.

4. The method for producing colored resin particles according to claim 3, wherein the polymerizable vinyl-based monomer contains (meth)acrylic acid ester having an alkylene oxide group.

5. The method for producing colored resin particles according to claim 3 or 4, wherein the step of coating with a polymer derived from a nitrogen-containing aromatic compound comprises dispersing the resin particles in an aqueous medium containing 0.5 to 2.0 molar equivalents of alkali metal salt of inorganic peracid with respect to the nitrogen-containing aromatic compound, thereby forming a dispersion, adding the nitrogen-containing aromatic compound to the dispersion, and stirring the resulting mixture, so as to coat the surfaces of the resin particles with the polymer of the nitrogen-containing aromatic compound.

6. A resin composition, containing the colored resin particles according to claim 1 or 2.

7. A molded body, obtained by molding the resin composition according to claim 6.

8. A light-diffusing plate, containing the colored resin particles according to claim 1 or 2.

9. A coating material, containing the colored resin particles according to claim 1 or 2.

10. A coated product, obtained by applying the coating material according to claim 9 to a base.

11. An optical film, obtained by applying the coating material according to claim 9 to a transparent base film.

12. A anti-glare film, obtained by applying the coating material according to claim 9 to a transparent base film.

13. An external preparation, containing the colored resin particles according to claim 1 or 2.

## Patentansprüche

1. Gefärbte Harzteilchen, die mit einem Polymer, das von einer stickstoffhaltigen aromatischen Verbindung abgeleitet ist, oberflächenbeschichtet sind, wobei jedes der Teilchen eine unregelmäßige Form mit einer kreisförmigen Erscheinung, wenn aus einer Richtung betrachtet, in welcher eine projizierte Fläche davon maximal wird, und mit einer nicht-kreisförmigen Erscheinung, wenn aus einer Richtung betrachtet, in welcher die projizierte Fläche davon minimal wird, aufweist, wobei:
die stickstoffhaltige aromatische Verbindung Pyrrol oder ein Pyrrolderivat ist;
die Harzteilchen irgendeine aus einer ausgesparten Form, einer halbkugelförmigen Form, einer pilzartigen Form oder einer doppelkonvexen linsenartigen Form aufweisen; und
die Harzteilchen mit der doppelkonvexen linsenartigen Form erhältlich sind indem Keimteilchen ermöglicht wird, ein polymerisierbares Monomer auf Vinylba-sis in einer wässrigen Emulsion zu absorbieren, wobei das polymerisierbare Monomer auf Vinylbasis 5 bis 50 Gew.-% eines polyfunktionalen Monomers enthält, in welchem ein Molekül zwei oder mehrere polymerisierbare Vinylgruppen aufweist, und durch das Polymerisieren des absorbierten polymerisierbaren Monomers auf Vinylbasis.

2. Gefärbte Harzteilchen nach Anspruch 1, wobei die Harzteilchen durch das Polymerisieren eines polymerisierbaren Monomers auf Vinylbasis, enthaltend (Meth)acrylsäureester mit einer Alkylenoxidgruppe, erhalten werden.

3. Verfahren zur Herstellung von gefärbten Harzteilchen, umfassend:
einen Schritt des Erhaltens von Harzteilchen, wobei jedes dieser eine unregelmäßige Form mit einer kreisförmigen Erscheinung, wenn aus einer Richtung betrachtet, in welcher eine projizierte Fläche davon maximal wird, und mit einer nicht-kreisförmigen Erscheinung, wenn aus einer Richtung betrachtet, in welcher die projizierte Fläche davon minimal wird, aufweist, durch das Polymerisieren eines polymerisierbaren Monomers auf Vinylbasis; und
einen Schritt des Beschichtens der Oberflächen der erhaltenen Harzteilchen mit einem Polymer, das von einer stickstoffhaltigen aromatischen Verbindung abgeleitet ist, wobei:
die stickstoffhaltige aromatische Verbindung Pyrrol oder ein Pyrrolderivat ist;
die Harzteilchen irgendeine aus einer ausgesparten Form, einer halbkugelförmigen Form, einer pilzartigen Form oder einer doppelkonvexen linsenartigen Form aufweisen; und
der Schritt des Erhaltens von Harzteilchen mit der doppelkonvexen linsenartigen Form das Ermöglichen, dass Keimteilchen ein polymerisierbares Monomer auf Vinylbasis in einer wässrigen Emulsion absorbieren, wobei das polymerisierbare Monomer auf Vinylbasis 5 bis 50 Gew.-% eines polyfunktionalen Monomers enthält, in welchem ein Molekül zwei oder mehrere polymerisierbare Vinylgruppen aufweist, und das Polymerisieren des absorbierten polymerisierbaren Monomers auf Vinylbasis umfasst.

4. Verfahren zur Herstellung von gefärbten Harzteilchen nach Anspruch 3, wobei das polymerisierbare Monomer auf Vinyibasis (Meth)acrylsäureester mit einer Alkylenoxidgruppe enthält.

5. Verfahren zur Herstellung von gefärbten Harzteilchen nach Anspruch 3 oder 4, wobei der Schritt des Beschichtens mit einem Polymer, das von einer stickstoffhaltigen aromatischen Verbindung abgeleitet ist, das Dispergieren der Harzteilchen in einem wässrigen Medium, enthaltend 0,5 bis 2,0 Moläquivalente eines Alkalimetallsalzes einer anorganischen Persäure in Bezug auf die stickstoffhaltige aromatische Verbindung, wodurch eine Dispersion gebildet wird, das Hinzufügen der stickstoffhaltigen aromatischen Verbindung zu der Dispersion und das Rühren der resultierenden Mischung umfasst, um die Oberflächen der Harzteilchen mit dem Polymer der stickstoffhaltigen aromatischen Verbindung zu beschichten.

6. Harzzusammensetzung, enthaltend die farbigen Harzteilchen nach Anspruch 1 oder 2.

7. Formkörper, erhalten durch das Formen der Harzzusammensetzung nach Anspruch 6.

8. Lichtdiffusionsplatte, enthaltend die gefärbten Harzteilchen nach Anspruch 1 oder 2.

9. Beschichtungsmaterial, enthaltend die gefärbten Harzteilchen nach Anspruch 1 oder 2.

10. Beschichtetes Produkt, erhalten durch das Aufbringen des Beschichtungsmaterials nach Anspruch 9 auf eine Basis.

11. Optischer Film, erhalten durch das Aufbringen des Beschichtungsmaterials nach Anspruch 9 auf einen transparenten Basisfilm.

12. Blendfreie Folie, erhalten durch das Aufbringen des Beschichtungsmaterials nach Anspruch 9 auf einen transparenten Basisfilm.

13. Externe Zubereitung, enthaltend die gefärbten Harzteilchen nach Anspruch 1 oder 2.

## Revendications

1. Particules de résine colorée enrobées superficiellement avec un polymère dérivé d'un composé aromatique azoté, dans lesquelles chacune des particules présente une forme irrégulière d'apparence circulaire lorsqu'elles sont observées depuis une direction dans laquelle une zone projetée de celle-ci devient maximale, et d'apparence non circulaire lorsqu'elles sont observées depuis une direction dans laquelle la zone projetée de celle-ci devient minimale, dans lesquelles:
le composé aromatique azoté est un pyrrole ou un dérivé du pyrrole;
les particules de résine ont une forme parmi une forme creusée, une forme hémisphérique, une forme de champignon ou une forme ressemblant à une double lentille convexe; et
les particules de résine ayant la forme ressemblant à une double lentille convexe peuvent s'obtenir en faisant que des particules d'ensemencement absorbent un monomère à base de vinyle polymérisable en émulsion aqueuse, le monomère à base de vinyle polymérisable contenant de 5 à 50% en poids d'un monomère polyfonctionnel dans lequel une molécule a deux groupements vinyle polymérisables ou plus, et en polymérisant le monomère à base de vinyle polymérisable absorbé.

2. Les particules de résine colorée selon la revendication 1, dans lesquelles les particules de résine sont obtenues par polymérisation d'un monomère à base de vinyle polymérisable contenant un ester d'acide (méth)acrylique présentant un groupement oxyde d'alkylène.

3. Un procédé de production de particules de résine colorée, comprenant:
une étape d'obtention des particules de résine, chacune d'entre elles présentant une forme irrégulière d'apparence circulaire lorsqu'elles sont observées depuis une direction dans laquelle une zone projetée de celle-ci devient maximale, et d'apparence non circulaire lorsqu'elles sont observées depuis une direction dans laquelle la zone projetée de celle-ci devient minimale, par polymérisation d'un monomère à base de vinyle polymérisable; et
une étape d'enrobage des surfaces des particules de résine obtenues avec un polymère dérivé d'un composé aromatique azoté, dans lequel:
le composé aromatique azoté est un pyrrole ou un dérivé du pyrrole;
les particules de résine ont une forme parmi une creusée, une forme hémisphérique, une forme de champignon ou une forme ressemblant à une double lentille convexe; et
l'étape d'obtention des particules de résine ayant la forme ressemblant à une double lentille convexe comprend permettre aux particules d'ensemencement d'absorber un monomère à base de vinyle polymérisable en émulsion aqueuse, le monomère à base de vinyle polymérisable contenant de 5 à 50% en poids d'un monomère polyfonctionnel dans lequel une molécule a deux groupements vinyle polymérisables ou plus, et en polymérisant le monomère à base de vinyle polymérisable absorbé.

4. Le procédé de production de particules de résine colorée selon la revendication 3, dans lequel le monomère à base de vinyle polymérisable contient un ester d'acide (méth)acrylique présentant un groupement oxyde d'alkylène.

5. Le procédé de production de particules de résine colorée selon la revendication 3 ou 4, dans lequel l'étape d'enrobage avec un polymère dérivé d'un composé aromatique azoté comprend la dispersion des particules de résine en milieu aqueux contenant de 0.5 à 2.0 équivalents molaires d'un sel de métal alcalin de peracide inorganique par rapport au composé aromatique azoté, formant ainsi une dispersion, l'addition du composé aromatique azoté à la dispersion et l'agitation du mélange obtenu, de manière à enrober les surfaces des particules de résine avec le polymère du composé aromatique azoté.

6. Une composition de résine, contenant les particules de résine colorée selon la revendication 1 ou 2.

7. Un corps moulé, obtenu par moulage de la composition de résine selon la revendication 6.

8. Une plaque à diffusion de lumière, contenant les particules de résine colorée selon la revendication 1 ou 2.

9. Une matière d'enrobage, contenant les particules de résine colorée selon la revendication 1 ou 2.

10. Un produit enrobé, obtenu par application de la matière d'enrobage selon la revendication 9 à une base.

11. Un film optique, obtenu par application de la matière d'enrobage selon la revendication 9 à un film de base transparent.

12. Un film antireflet, obtenu par application de la matière d'enrobage selon la revendication 9 à un film de base transparent.

13. Une préparation externe, contenant les particules de résine colorée selon la revendication 1 ou 2.
